# EUROPEAN PATENT SPECIFICATION

(11) **EP 0 569 879 B1**
(45) Date of publication and mention of the grant of the patent: **17.04.2002**
(21) Application number: 93107453.8
(22) Date of filing: 07.05.1993
(51) Int. Cl.: A61B 5/22, A63B 21/00

(54) **Exercise system for managing physiological intensity of exercise**
Übungssystem zur Überwachung des physiologischen Übungsgrades
Système d'entraînement de surveillance de l'intensité physiologique de l'entraînement

(30) Priority: 12.05.1992 US 881918; 03.11.1992 US 971422
(43) Date of publication of application: 18.11.1993
(60) Divisional application: 00107164.6
(73) Proprietor: LIFE FITNESS, Franklin Park, Illinois 60131 (US)
(72) Inventor: Golen, Emil S., Barrington, Illinois 60010 (US); Oglesby, Gary E., Manhattan, Illinois 60442 (US); Alexander, Donald J., Milwaukee, Wisconsin 53217 (US); Leon, Tomas F., Chicago, Illinois 60660 (US); Quast, Robert E., Kildeer, Illinois 60047 (US); Hood, Robert L., Grayslake, Illinois 60030 (US); Thum, David J., Roselle, Illinois 60172 (US)
(74) Representative: Lorenz, Werner, Dipl.-Ing.

(56) References cited:
- GB-A- 2 187 554
- US-A- 3 395 698
- US-A- 4 378 111
- US-A- 4 817 938
- US-A- 4 898 182
- US-A- 5 207 621

## Description

### Field of the Invention

This invention relates to aerobic exercise equipment, and more particularly to exercise equipment with loads which are variable for maintaining a user's heart rate or other physiological condition at a predetermined level.

### Background of the Invention

Exercise generally, and aerobic exercise in particular, is of value to individuals because it conditions and improves respiratory and circulatory systems. Exercise is characterized in part by intensity and duration. Intensity, which may be thought of as the effort expended by an individual, is reflected in the individual's physiological condition. For example, heart rate, breathing and metabolism increase with exercise intensity, and are referred to herein as the "physiological indicators of intensity".

While exercise intensity is a physiological phenomenon and is properly measured by one of the physiological indicators, it is also manifested outside the body by an individual's physical movement or by the physically measurable work performed by the individual. For example, at a fixed level of resistance, pedal rpm on stationary exercise bicycle gives some indication of the individual's level of exercise intensity. This type of indication is referred to herein as an externally observable "physical indicator of intensity".

While physical indicators of intensity such as ergometers or tachometers are easier to implement than physiological indicators of intensity, their usefulness is limited because of the subjective nature of exercise. For example, two persons riding a stationary bicycle at the same levels of speed and resistance would appear to have the same level of exercise intensity based externally observable physical indicators, such as pedal rpm. Depending upon each person's level of fitness, their respective actual levels of exercise intensity (as measured by one of the physiological indicators) could be quite different.

For effective aerobic exercise, it is necessary that physiological intensity reach a certain minimum threshold. At the same time, if exercise is too intense, it becomes primarily anaerobic (i.e., oxygen depleting). Exercise at an excessive level of intensity does not yield additional improvements in the body's aerobic fitness.

Thus, between the upper and lower thresholds of training intensity lies the aerobic training range. It is important, therefore, to monitor levels of intensity to ensure that intensity falls within this training range. To monitor intensity, a number of physiological conditions may be inspected, including heart rate, breathing as a percentage of maximal oxygen intake, and metabolism.

Typically, such monitoring requires a sensor which is placed in physical contact with an individual to measure the individual's heart rate or the like. The individual can then be apprised of his or her level of exercise intensity. One simple example of this is someone on a stationary exercise bicycle who takes his or her own pulse after a workout to determine whether he or she has reached a sufficient level of aerobic intensity.

A more sophisticated approach, however, is to employ biofeedback techniques for periodically adjusting workout intensity in response to the physiological indicators of intensity, such as heart rate. Examples of such devices are provided in U.S. Patents Nos. 3,395,698 and 3,744,480. While theoretically any of the physiological indicators may be used, it is most practical to use heart rate, and therefore the examples set forth herein all use heart rate as the physiological indicator of intensity.

Devices which employ biofeedback techniques include exercise bicycles having variable load resistance to pedal movement. This resistance can be provided by well-known mechanical and electrical devices, including alternators, which can be coupled by chains or belts to the pedals.

In such devices, a heart rate detector is coupled to the user, typically by an ear clip. A target heart rate is selected, either by the user or automatically by the device. As the user exercises, his or her pulse is periodically measured and compared to the target heart rate. If the user's heart rate is below the target, the load resistance is increased. Likewise, if the user's heart rate is above the target heart rate, the load resistance is decreased.

In this manner, these exercise devices function as "biofeedback-type systems". They adjust load resistance as a function of heart rate to establish and maintain the user's heart rate (i.e. physiological exercise intensity) at or near the desired or target level. For a variety of reasons, these devices have been less than optimal.

Specifically, for effective operation, these systems depend on the continuous availability of heart rate data. For example, if the user wears an ear clip, heart rate data could be available throughout the exercise.

There have, however, been recent advances in heart rate detection technology, such as disclosed in US-A-5 243 993 (assigned to the assignee of this application). Unlike older heart rate detection techniques which required cumbersome ear clips or the like, this new detection technology measures a user's heart rate whenever his or her hands are placed on the exercise device's handgrips. Such handgrips, for example, can be located on the handlebars of an exercise bicycle.

One disadvantage, however, of locating the detectors on the handgrips in a heart rate measurement system is that the user will tend to remove his hands from the handgrips from time to time. When the user's hands are removed, the biofeedback type device will receive no information about the user's heart rate. To be practical, a heart rate management system should continue to operate effectively even when the flow of heart rate data is interrupted for periods as long as ninety seconds. Existing systems are not designed to handle intermittent heart rate signals.

Moreover, it has come to be appreciated that systems should not only be able to process intermittent heart rate data, but in fact periodically should also invite users to remove their hands from the handgrips (or otherwise disengage the sensor). In this manner, the user does not feel "chained" to the heart rate measuring device, and is free to wipe his or her brow, turn the pages of a book, adjust a personal tape player, or do any of the many things a person riding an exercise bicycle is likely to do to divert their attention from an otherwise boring exercise.

As explained above, externally observable indicators of exercise intensity (such as pedal speed) are related to the user's actual physiological level of exercise intensity, but are of limited value as tools for measuring that intensity because they are not calibrated for each individual user. Consequently, biofeedback-type devices of the past have relied on heart rate, and have not utilized external indicators (such as pedal rpm) in conjunction with physiological data to attain the highest possible performance.

Also, in some existing devices, load changes tend to be too abrupt and too frequent. Preferably, for most people load changes should be gradual. On the other hand, it may be desirable to make load changes more dramatic for persons in better physical condition. It is also desirable that the device should anticipate changes in the user's heart rate so that load can be adjusted earlier, and therefore more gradually.

Stair climbing is recognized as a particularly effective type of aerobic exercise, and as a result, exercise machines facilitating this type of exercise are becoming increasingly popular.

There have been a variety of approaches taken in designing stair climbing apparatus, including the simulation of an actual staircase as illustrated in U.S. Patent Nos. 3,497,215 and 4,687,195. Another approach has been to simulate the action of stair climbing by using a pair of reciprocating pedals.

An example of the later approach is described in U.S. Patent No. 5,135,447 issued to Robards, Jr. et al. on August 4, 1992. Other examples of this type of machine are described in U.S. Patent Nos. 3,316,819, 3,529,474, 3,628,791, 3,979,302, 4,496,147, 4,600,187, 4,676,501, and 4,720,093.

As exemplified by U.S. Patent No. 5,135,447, these machines include a pair of pedals which are adapted for vertical reciprocating motion to provide a user who is standing on the pedals with a simulated climbing exercise. The vertical reciprocating motion is typically translated into a rotary motion by a suitable system of belts, gears and clutches, for example.

The rotary motion (which may be imparted to a shaft, flywheel or the like) is opposed by a variable source of resistance force, typically an alternator, eddy current break or the like. The alternator is responsive to a control signal for selectively varying the level of resistance. when the user's rate of exercise reaches a predetermined threshold, the control signal causes the resistance to sharply increase. The higher resistance compels the user to decrease his or her rate of exercise. In this manner, variable resistance devices such as alternators have been used to control the rate of user exercise. The rate of user exercise is also controlled by prompting the user to pedal faster or slower. Consequently, variations in exercise intensity are achieved by varying the rate at which the user steps.

US 4,898,182 discloses an exercise apparatus acquiring information about a user to set a target level for exercise intensity.

In general, the objective of these systems is to simulate stair climbing. Stair climbing is characterized by its uniform, repetitive nature. Ideally, stair climbing apparatus would provide a more dynamic climbing simulation to increase user interest.

### Summary of the Invention

One object of the invention is to provide an exercise apparatus for managing the physiological intensity of exercise. The apparatus is not only very effective, but also allows the user the comfort and convenience of being able to periodically disengage himself or herself from the pulse sensor and to exercise at various speeds. Moreover, devices built in accordance with the invention continue to operate effectively even when the signal representing heart rate is interrupted for periods as long as ninety seconds. The invention uses externally observable physical indicators of exercise intensity such as pedal rpm in conjunction with physiological indicators such as heart rate for improved control over exercise intensity.

In one embodiment of the invention, an exercise apparatus such as a stationary bicycle is provided for establishing and maintaining a user's heart rate near a target heart rate during exercise. The apparatus includes pedals or other suitable members which are manipulated by the user in an exercise movement. A load device opposes the movement of the pedals with selectable levels of resistance. Thus, the difficulty of pedaling is adjustable to vary the intensity of exercise.

A sensor, preferably located on the bicycle's handlebars, detects the user's heart rate, while a tachometer measures the speed at which the user pedals. An internal computer or other suitable control circuit is connected to the sensor, tachometer and load device. A control panel, also connected to the computer, provides the user with a display of information, including heart rate, rpm and load level. A keyboard on the control panel enables the user to enter information such as his or her age or desired target heart rate.

During the initial portion of the exercise, the computer reads the user's heart rate, and adjusts the load device to make pedaling harder or easier in order to establish and maintain the user's heart rate near the target level. For example, if the user's heart rate was below the target heart rate and was not increasing, then the computer would adjust the load device to make pedaling more difficult, thereby tending to increase the user's heart rate.

Once the user has attained the target heart rate, the computer invites the user from time to time to disengage the sensor by taking his or her hands off the handlebars for a limited time period. This allows the user freedom to use his or her hands during exercise without being "chained" to the sensors. The time limit which the user is allowed to keep his or her hands off the sensors is determined by the computer based on recent changes in the user's heart rate.

When the user's hands are removed from the sensors, the computer continues to maintain the user's heart rate near the target based on changes in the user's rpm level. In this manner, the computer uses pedal rpm (a physical indicator of exercise intensity) to supplement heart rate (a physiological indicator of exercise intensity). The computer adjusts load resistance in accordance with pedal rpm to maintain the user's heart rate near the target.

Specifically, if the user pedals faster over time, the computer reduces the load resistance to anticipate the increase in the user's heart rate. Conversely, if the user pedals slower, the computer increases load resistance to anticipate the decrease in the user's heart rate.

As discussed above, rpm alone does not provide a valid indication of an individual user's true level of physiological intensity. We have realized, however, that externally observable physical indicators such as rpm do have value in managing physiological exercise intensity when used in conjunction with the physiological indicators such as heart rate.

Thus, once the heart rate sensor indicates that the user has attained the target heart rate, the computer uses pedal rpm to supplement information received from the heart rate sensor, particularly during times when heart rate data is unavailable (such as when the user's hands are off of the heart rate sensors). Even when the user's hands are on the sensors, the computer uses pedal rpm to anticipate changes in heart rate. By anticipating changes in heart rate, load adjustments are made more gradual, and the heart rate more steady. When the computer requires fresh heart rate data, it prompts the user via a visual display to place his or her hands on the sensors. If the user fails to do so within a predetermined amount of time, the computer initiates a warning signal, such as a bell. If the user continues to ignore the prompt, the computer substantially reduces the load resistance. This encourages the user to place his hands on the sensors, as well as deters the user from exercising beyond his aerobic level.

The invention may also be embodied in a kit comprising sensors, controllers and computers, which could be sold separably for upgrading existing exercise devices.

It is, therefore, an object of the invention to provide an exercise intensity management system which does not require the user to remain constantly coupled to a heart rate measuring device. Moreover, the system actually invites the user to release or disengage the measuring device, and then per-iodically prompts the user to re-engage the measuring device as necessary.

It is an additional object of the present invention to provide an exercise management system which adjusts the load resistance of the exercise device in response to changes in the user's rate of exercise (or other externally observable physical indicator) as well as the user's heart rate.

A preferred object of the invention is to provide an exercise apparatus for dynamically simulating hill climbing exercise.

In accordance with this preferred embodiment of the invention, a climbing exercise apparatus is provided having a frame on which two pedals are pivotally mounted to provide a user with a vertically reciprocating exercise movement, and a control panel for user input and output. A dynamic break, alternator or the like applies a variable level of resistance (via a suitable transmission) against the user's movement. The resistance automatically varies over time in accordance with a control signal generated by a computer. The value of the control signal is independent of the rate at which the user steps.

In this preferred embodiment of the invention, a computer or other control circuit successively selects a series of difficulty values which correspond to hill heights, sizes, grades or like measures of difficulty. A primary display successively displays images of hills. Each displayed hill image is associated with the currently selected one of the series of difficulty values and that value is graphically represented as part of or in visual association with the hill image.

For example, in the preferred embodiment, an LED display depicts successive hills by lighting a vertical column of LEDS. The value or "height" of the hill is represented by the number of LEDs that are illuminated in the column. Alternatively, more sophisticated representations may be employed, including video depictions of hills.

As each hill is displayed, a control circuit generates a control signal to vary the load resistance against the user as a function of the displayed hill's difficulty value. In this manner, the exercise movement gets more difficult as the hills depicted by the primary display appear "higher".
In the preferred embodiment, the control signal is a pulse train. Each pulse is generated at regular intervals of approximately 50 milliseconds. The width (or "duty cycle") of each pulse, however, is modulated by the computer, to effectively control the resistance load imparted by the alternator against the user's movement.

The apparatus may also include a keypad or the like for entering data such as the user's weight and/or an effort level that is selected from a predetermined range. In accordance with one preferred aspect of the invention, the duty cycle of the control signal is modulated as a function of the displayed hill's difficulty value, the square of the user's weight and the user-entered effort level.

### Brief Description of the Drawings

FIG. 1 is a perspective view of an exercise bicycle in accordance with one embodiment of the invention;
FIG. 2 is a perspective view of an exercise treadmill in accordance with one embodiment of the invention;
FIG. 3 is a perspective view of an exercise machine for simulating stair climbing in accordance with one embodiment of the invention;
FIG. 4 is a generalized block diagram of an exercise device in accordance with the invention;
FIG. 5 is an illustration of a control and display panel in accordance with one embodiment of the invention;
FIGS. 6 through 15 are logic flow charts of the software program operating in the memory of the microcomputer of FIG. 4 which performs the functions specified by the invention;
FIG. 16 is a perspective view of a climbing-type exercise device in accordance with a second embodiment of the invention;
FIG. 17 is a block diagram of the exercise device shown in FIG. 16;
FIG. 18 is a plan view of a control panel that is part of the exercise device illustrated in FIG. 16; and
FIGS. 19 and 20 are logic flow charts illustrating the operation of the exercise device shown in FIG. 16.

### Detailed Description of the Invention

### A. Technical Environment

In the present invention, a biofeedback-type heart rate management system is provided which provides new, useful features and superior performance.

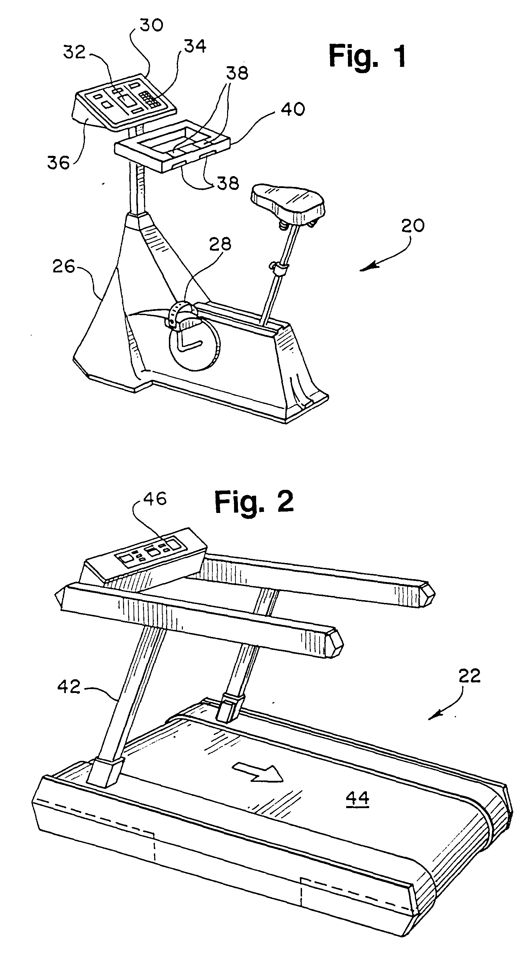

Referring to FIGS. 1, 2 and 3, computer-controlled exercise devices 20, 22, and 24 employing one embodiment of the invention are illustrated. Except for the aspects of the invention described herein, each of the devices 20, 22 and 24 is well-known and commercially available from suppliers such as the Life Fitness of Franklin Park, Illinois.

The device 20 is a typical exercise bicycle, and includes a frame 26 on which a user may sit. The user exercises by manipulating pedals 28 in an exercise movement. A resistance load device (not illustrated in FIG. 1) is housed within the frame 26 and is coupled to the pedals 28 by a belt, chain or the like. The load device provides a selectively variable resistance to the movement of the pedals 28. The load device may be mechanical or electromechanical. One example of such a device is provided in the U.S. Patent No. 4,817,938 issued April 4, 1989.

A control panel 30 includes a data display 32 and a keypad 34 which enables communication between an internal computer and the user. The internal computer generates a load control signal, which is coupled to the variable load resistance device. Thus, a user may select a particular load level via the keypad 34. The internal computer then generates the appropriate load control signal.

The bicycle 20 also includes a heart rate monitor 36, which is preferably the heart rate detection system disclosed in US-A-5 243 993. The monitor 36 includes four electrodes or "biopotential sensors" 38 which are mounted on a handlebar 40 of the bicycle 20. To engage the monitor 36, a user riding the bicycle 20 touches the electrodes 38 with the palms and fingers of his hands.

Referring to FIG. 2, the device 22 is a typical treadmill, which includes a frame 42 having an endless belt 44 upon which a user runs or walks. The movement of the belt 44 serves the same function as the movement of pedals 28 in bicycle 20. The treadmill 22 also includes a motor which is housed within the frame 42 and is coupled to belt 44. The motor, under computer control, drives the belt 44 at a predetermined rate. One example of such a device is provided in US-A-5 382 207 and assigned to the assignee of this application.

As described above in connection with the bicycle 20, the treadmill 22 also includes a control panel 46 which enables communication between a computer and the user. The computer or user generates a speed control signal that controls the speed of the belt 44 of the treadmill 22. The computer also generates an incline control signal which allows the belt 44 to be selectively placed on an incline relative to the horizontal. The steeper the incline, the more intense a user's exercise at a given belt speed. Thus, the incline functions as a load resistance device for providing a selectively variable resistance to the user's exercise movement. The treadmill 22 also includes a heart rate monitor such as the heart rate monitor 36 of the bicycle 20 (not illustrated).

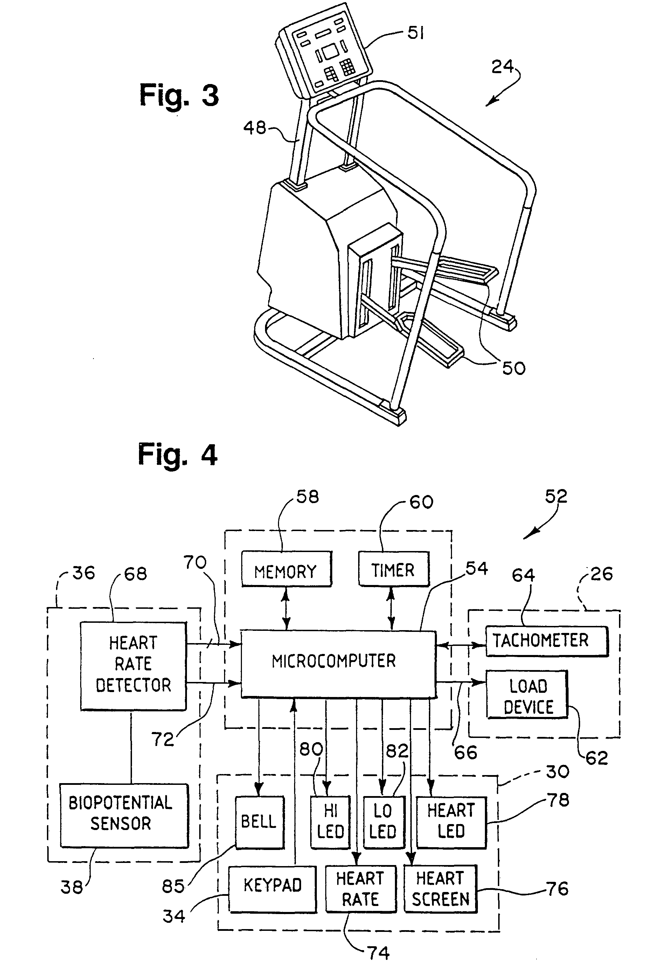

Referring to FIG. 3, device 24 is a typical stair climbing simulating machine which includes a frame 48 having vertically reciprocating pedals 50 upon which a user stands to simulate an aerobic stair climbing movement. One example of such a device is provided in US-A-5 238 462 and assigned to the assignee of this application. The movement of the pedals 50 serves a comparable function as the movement of the pedals 28 in bicycle 20 and belt 44 in treadmill 22. The stair machine 24 also includes a resistance load device which is housed within the frame 48 and is coupled to the pedals 50. The load device provides a selectively variable resistance to the movement of the pedals 50. The load device may be mechanical or electromechanical, and functions to increase the user's exercise intensity. Alternatively, the load device could vary the vertical distance which the pedals 50 reciprocate to provide greater exertion per step.

As described above in connection with the bicycle 20, the stair climbing machine 24 also includes a control panel 51 which enables communication between an internal computer rand the user. The computer generates a load control signal, which is coupled to the variable load resistance device. The device 24 also includes a heart rate monitor (not illustrated in FIG. 3) such as the heart rate sensor monitor 36 of the bicycle 20.

As will be apparent, biofeedback-type devices employing the present invention may take a number of forms, including that of each of the devices 20, 22 and 24. Each of these devices includes a movable member such as pedals 28 or belt 44 which provides the user with an exercise movement The manipulation can be by hand or foot, and may be circular, reciprocating and so forth. Each device also includes a mechanism for controlling the rate or resistance of the exercise. This mechanism is selectively adjustable to vary the exercise intensity experienced by the user. Throughout this application, we refer to such devices as load devices, but it will be understood that the term load device contemplates any apparatus (such as those described above) which can be used to increase or decrease the intensity of the user's exercise.

Each device 20, 22 and 24 also includes a pulse monitor along with analog or digital processing circuits for comparing the heart rate measured by the pulse sensor to a predetermined or target heart rate. As explained above, heart rate is but one of the physiological indicators of exercise intensity, and for convenience it is used in the foregoing illustrations. It should be understood that the monitors could measure other physiological indicators.

Finally, each device 20, 22, and 24 is equipped with a rate sensor (not shown in FIGS. 1-3) measuring rate of exercise activity (e.g. pedal rpm). It will be recalled from the Background discussion that phenomenon such as pedal speed which are observable outside the body are some indication of the user's exercise intensity, and are referred to as externally observable physical indicators of intensity. They can be thought of as rough approximators of true physiological intensity, which is best measured by a physiological indicator such as heart rate.

Because exercise movements in the devices 20, 22 and 24 tend to be repetitive, it is helpful to describe these movements in terms of revolutions or cycles per second. For example, on the exercise bicycle 20, the user exercises by rotating the pedals 28. The rate of exercise is simply the rate at which the pedals 28 rotate, and is expressed in revolutions per minute (rpm). It will be understood that references herein to rpm contemplate not only any measure of the cycles per second of repetitive exercise movements (such as stair climbing) but also the broader concept of externally observable physical indicators of exercise intensity.

### B. General Operation of the Invention

For simplicity, the operation of the invention is explained with respect only to the bicycle 22. It is understood that in bicycle 20 pedal rpm is used as the externally observable physical indicator of exercise intensity. In accordance with the invention, bicycle 20 maintains the user's heart rate (or other physiological indicator of exercise intensity) near a target level by adjusting load resistance in response to the user's heart rate. The bicycle 20 can maintain the user's heart rate near the target level even when heart rate data is unavailable for limited time periods. Once the user has reached the target heart rate, the bicycle 20 also uses pedal rpm to supplement the information it receives about heart rate.

In the preferred embodiment, the user begins an exercise session by entering his age on the keyboard 34 of control panel 30. The system then computes a target heart rate based on the user's age in accordance with any suitable formula. Alternatively, the user may designate a target heart rate. The user then enters an initial load level.

The bicycle 20 then sets load resistance at the user-selected load level for a three-minute warm-up period. At the conclusion of the warm-up period, the bicycle 20 prompts the user with data display 32 to place his hands on the heart rate sensors 38. When the user's hands are placed on the sensors 38, the user's heart rate is displayed on the data display 32, and the heart rate monitor 36 begins to periodically sample the user's heart rate. Bicycle 20 adjusts the load in accordance with the user's heart rate to establish the user's heart rate near the target.

Once the user has reached or exceeded the target heart rate, the bicycle 20 begins to use pedal rpm (physical indicators of exercise intensity) to supplement heart rate (a physiological indicator of exercise intensity). Thus, if the user increases pedal rpm, the bicycle 20 reduces load resistance to anticipate the user's increase in heart rate. Conversely, if the user decreases pedal rpm, the bicycle 20 increases load resistance to anticipate the user's decreasing heart rate.

As discussed above, physically-based indicators of exercise intensity (such as pedal rpm) do not by themselves provide valid indications of an individual user's true level of physiological intensity. We have realized, however, that physically-based indicators do have value in managing physiological exercise intensity when used in conjunction with the physiological indicators.

In the case of bicycle 20, heart rate is used to measure physiological exercise intensity, and pedal rpm is used to measure physical level of exercise intensity. Once the user has reached the target heart rate, the user's heart rate is closely related to the user's pedal rpm. In effect, what was formerly data of limited value (i.e. pedal rpm), is now very useful because it has been associated with user's true physiological condition (i.e. heart rate).

Thus, information provided by the pedal rpm can be effectively used to supplement information provided by heart rate. Because the bicycle's 20 control system has two sources of information about the user's exercise intensity, its performance is improved. If heart rate data is temporarily unavailable, changes in pedal rpm can be used to adjust exercise load to maintain a constant heart rate. Moreover, changes in rpm (i.e. physical or external intensity) tend to anticipate changes in heart rate (i.e. true physiological intensity). By responding to the changes in rpm, load adjustments are made more gradual and heart rate more steady.

Until the user has reached the target heart rate, the bicycle 20 seeks to continually monitor the user's heart rate. Once the user has reached the target heart rate, however, the bicycle 20 will allow (or, preferably, invite) the user to remove his hands from the sensors 38 for periods of time such as ninety seconds. When the bicycle 20 requires fresh heart rate data, it prompts the user by means of the data display 32. The user then places his hands on the sensors 38 so that the bicycle 20 can take a new sample of heart rate. If the user's heart rate is at an appropriate level, the user is again invited to remove his hands from the sensors 38. This cycle is repeated throughout the exercise.

If the system prompts the user to place his hands on the sensors 38, and the user ignores the prompt for more then forty-five seconds, a bell or beeper is activated. If the user continues to ignore the prompt for an additional fifteen seconds, the load resistance is substantially reduced. It will be observed that during periods where the user's heart rate data is unavailable, the system can use rpm data to maintain the target heart rate.

### c. Hardware Description

FIG. 4 15 a generalized block diagram of a system 52 in accordance with the preferred embodiment of the invention which is used to implement the foregoing operations of the invention. The system 52 is illustrated herein as part of exercise bicycle 20. Of course, system 52 can be readily implemented in any of the exercise devices 20, 22 and 24, or in any other type of device providing a variable load resistance or speed control for exercise movement.

Referring to FIG. 4, the hardware elements of system 54 are illustrated. A microcomputer 54 controls system 52 and includes a memory 58 and a timer 60. In practice, the memory 58 should include both random access memory as well as read only or non-volatile memory for permanently storing the software programs which enable the microcomputer 54 to perform in accordance with the invention. The microcomputer 54 can be any suitable device such as the Motorola 68HC05. The microcomputer 54 communicates with a user via the control panel 30 (illustrated in FIG. 4 by dotted lines), which is described below in greater detail.

The microcomputer 54 controls a load device 62 mounted in the frame 26 (illustrated here by dotted lines). As described above, the load device 62 is operatively associated with the pedals 28 of the system 52 to provide a selectively variable resistance load against the exercise movement of the pedals 28 by the user. A device 64 for measuring the rate of exercise, which is preferably a tachometer, measures the rate of rotation of the pedals 28, and is accessible to the microcomputer 54 by a conventional input/output port. In some embodiments where the load device 62 is an alternator, the tachometer 64 can be implemented by simply measuring the frequency of the output of tile alternator. It will be observed that the tachometer 64 measures an externally observable physical indicator of tile user's exercise intensity (namely pedal rpm).

It will be apparent to those skilled in the art of microcomputers that input/output interface circuitry may be necessary to enable communication between microcomputer 54 and external devices 62 or 64. The exact nature of this interface circuitry will vary depending on the hardware which is used to implement the invention.

Preferably, the interface between the microcomputer 54 and the load device 62 should allow the microcomputer 54 to simply write an 8 bit number of between 0 and 250 to an output port 66. The number corresponds to one of 251 evenly-spaced graduations of load level resistance covering the working range of the load device 62. In this manner, the output of the microprocessor 54 to the port 66 that is used to control the load device 62 can be viewed as a load signal. From a programming prospective, the load signal can be represented by the contents of a location in the memory 58. A driver routine is then periodically called by an interrupt to write the stored value of load resistance to the port 66.

Of course, there are innumerable ways in which a microcomputer and a load device can be interfaced, and the present invention contemplates all such alternatives to the extent that they allow the microcomputer 54 to selectively adjust the resistance level of load device 62.

The microcomputer 54 provides a convenient and practical method of implementing the invention. It will be apparent to those skilled in the art that the logical functions necessary for carrying out the invention could also be implemented using other types of digital and analog circuitry.

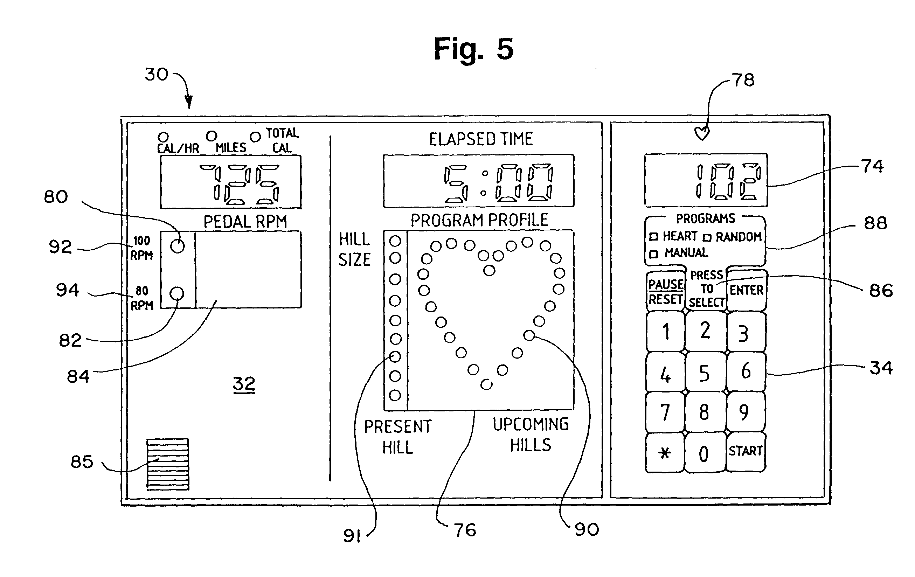

The system 52 also includes the heart rate monitor 36 illustrated in FIG. 5 by a dotted line. The monitor 36 includes a pulse sensor, which may be the electrodes 38 shown in FIG. 1, and a heart rate detector 68. There are numerous commercially available systems for measuring heart rate, but as indicated above, the preferred system is disclosed in US-A-5 243 993.

The heart rate detector 68 amplifies and filters the pulse signal received by the sensor 38. The pulse signal may undergo digital signal processing using circuitry within the heart rate monitor 36, or in microprocessor 54 itself. To simply this illustration, it is assumed that all processing of pulse signal takes place within heart rate monitor 36, which in turn provides an 8 bit value of heart rate over a line 70, and a single bit (the "engagement signal") over a line 72 indicating whether the user is currently engaged with the sensor.

In some systems, the engagement signal such as described above may be unavailable. As an alternative, the system 52 can assume that the user is disengaged from the monitor (i.e. by removing his hands, removing an ear clip, or otherwise interrupting the flow of heart rate data) whenever the monitor fails to deliver a heart rate within the range of 50 to 200.

FIG. 5 is a diagram of a control panel which, for the purposes of illustration, is the control panel 30 shown in FIG. 1. The control panel 30 interfaces with microcomputer 54 in any suitable manner such as by serial or parallel port. Because in practice the microcomputer 54 will control other functions of bicycle 20 in addition to managing heart rate, other features including an elapsed time display and a caloric consumption display are shown in FIG. 5. Contrastingly, in FIG. 4 only selected elements of the control panel 30 are illustrated.

For clarity, communication signals between microcomputer 54 and various elements of the control panel 30 are illustrated in FIG. 4 by separate arrows. In practice, suitable input/output interface circuitry can be used to facilitate communication between the microcomputer 54 and the control panel 30. As indicated, such communication might involve a serial or parallel link.

The control panel 30 includes the keypad 34, an LED heart rate display 74, an LED heart screen 76, an LED hands-on indicator 78, a high rpm LED 80, a low rpm LED 82, an LED rpm display 84, and an alarm bell 85. The specific configuration of the control panel 30, including its layout and the protocol used to communicate with the user are described in connection with the preferred embodiment of the invention as implemented in the exercise system 52. There is an infinite variety of audio and visual designs and techniques for establishing communication between the microcomputer 54 and the user, and the present invention contemplates the use of any suitable design or technique which achieves the functionality specified by the invention.

The keypad 34 is used by the user to communicate with the microcomputer 54. Conventionally, a computer controlled exercise bicycle 20 has several modes of operation. Thus, an existing program or mode of operation might allow the user to pedal at a constant level of load resistance. Another mode might enable the user to pedal at randomly selected levels of resistance. In the illustrated embodiment, the present invention is described as an additional mode of operation which can be referred to as a Heart Rate Management Mode.

The various modes of operation, including the novel Heart Rate Management Mode, may be selected by pressing a select key 86. Each time the select key 86 is pressed, one of the possible modes is selected. A panel 88 above the select key 86 displays indicia of each of the available modes. In the illustration, the available modes are depicted by the indicia "Random", "Manual" and "Heart [Rate Management]". These modes are illustrated solely as examples. A small LED next to each indicia indicates when the mode corresponding to the indicia has been selected.

The LED heart rate display 74 displays the current measured value of the user's heart rate when the user has placed his or her hands on the sensors 38. If, for reasons explained below, the user has engaged the sensors 38 but valid heart rate data is not available, then "Hr" or other symbol is displayed in the LED heart rate display 74 to signify that no valid data is yet available.

The heart-shaped LED hands-on indicator 78 is lit by the microcomputer 54 whenever the heart rate monitor 36 signals on the line 72 that the user has engaged the sensors 38. In this case, such engagement is achieved by the user placing his or her hands on the sensors 38. For convenience, we refer to this engagement as a hands-on condition, although it is to be understood that other types of engagement (such as by ear clip) are contemplated by the invention.

If the heart monitor 36 signals that the user's hands have engaged sensors 38, microcomputer 54 enables the heart-shaped LED hands-on indicator 78. Generally, the heart-shaped LED hands-on indicator 78 is lit concurrently with the display of heart rate data (or the "Hr" symbol, as the case may be) in the LED heart rate display 74.

The LED heart screen 76 is preferably an array of LEDs on which a heart 90 or other suitable symbol can be displayed. A row 91 of LED's can be used as a meter to display the relative value of load resistance. As will be explained, the heart symbol 90 is displayed by microcomputer 54 during those times when the user has his or her hands on the sensors 38. When the heart symbol is not displayed, the user is free to remove his hands from the sensors 38. If the microcomputer 54 determines that the user's current heart rate data is required, and the user's hands are not on the sensors, then the microcomputer 54 can flash the heart symbol to advise the user to place his or her hands on the sensors 38.

The LED rpm display 84 displays the current rpm or other measure of the rate of exercise movement performed by the user. The high rpm LED 80 and low rpm LED 82 are used to prompt the user to pedal at predetermined high and low rpm levels, respectively. Indicia 92 and 94 next to the high and low rpm LEDs 80 and 82, respectively, indicate that the predetermined high and low rpm levels are 100 and 80 rpm, respectively.

### D. Software Description

### 1. Main Routine

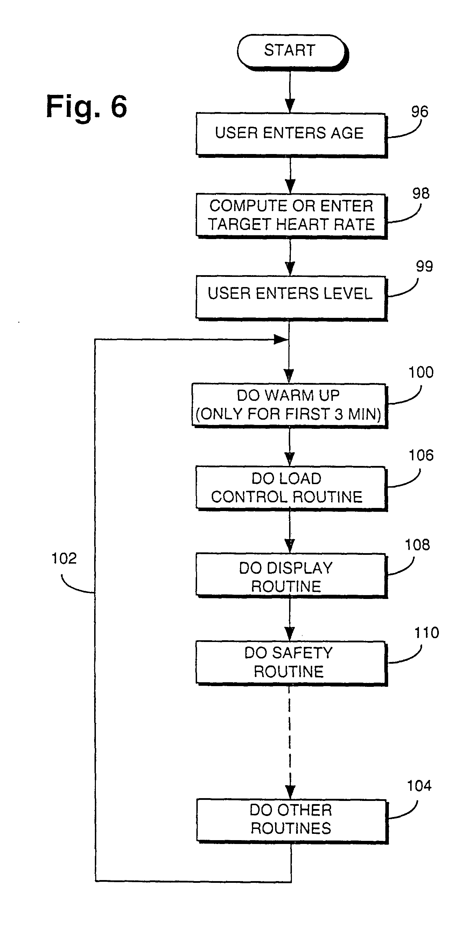

The software routines which enable the foregoing operation of the invention are resident in the memory 58 of microcomputer 54. Referring to FIGS. 6-15, logic flow charts of the software resident in memory 58 are provided. For convenience, selected variable names used in FIGS. 6-15 are included in parentheses throughout this specification. The main routine which calls the other major software modules is illustrated in FIG. 6. Beginning at a block 96, the microcomputer 54 prompts the user to enter his age (AGE). The microprocessor may use the LED heart rate display 74 for prompting, and the user may use the keypad 34 for data entry.

At a block 98, microcomputer 54 computes a target heart rate in accordance with a predetermined formula. A number of formulae are widely known. In the preferred embodiment, the target heart rate (HR∼TGT) is set to:$\text{HR_TGT = 220 - AGE * 0.7}$

Alternatively, the user may enter a target heart rate of his or her own selection.

At a block 99 the user enters a desired initial exercise load resistance level (LEVEL). Typically the range of possible exercise levels is scaled from 0 to 12.

At a block 100, the microcomputer enters a main driver loop 102 in which the major software modules implementing the invention in the system 52 are called. It will be noted that the software resident in the memory 58 may include other functions not related to the invention. These functions may be executed in the main driver loop 102, as indicated by a block 104.

At the block 100, the user is prompted to ride the bicycle 20 for a predetermined warm up period such as three minutes during which time the load level will be established and maintained the constant value LEVEL which the user selected at the block 99. After completion of the three minute warm-up, the warm-up routine 100 is suppressed and is no longer called during each iteration of main driver loop 102.
As discussed in more detail below, the major software elements implementing the invention are the load control module shown in a block 106, the display module shown in a block 108, and the safety module shown in block 110. Depending on the execution speed of the microcomputer 54, a number of iterations of main driver loop 102 will be completed each second. In practice, it is desirable to execute the load control module 106 about once each second. The display and safety modules 108 and 110 may be executed more frequently. An interrupt is triggered once per second and sets a flag (not shown). The load control module 106 is called from the main driver loop 102 only when this flag is set. It will be noted that during the three-minute warm-up, the load control routine is suppressed and is not called with each iteration of the main driver loop 102.

### 2. Load Control Module

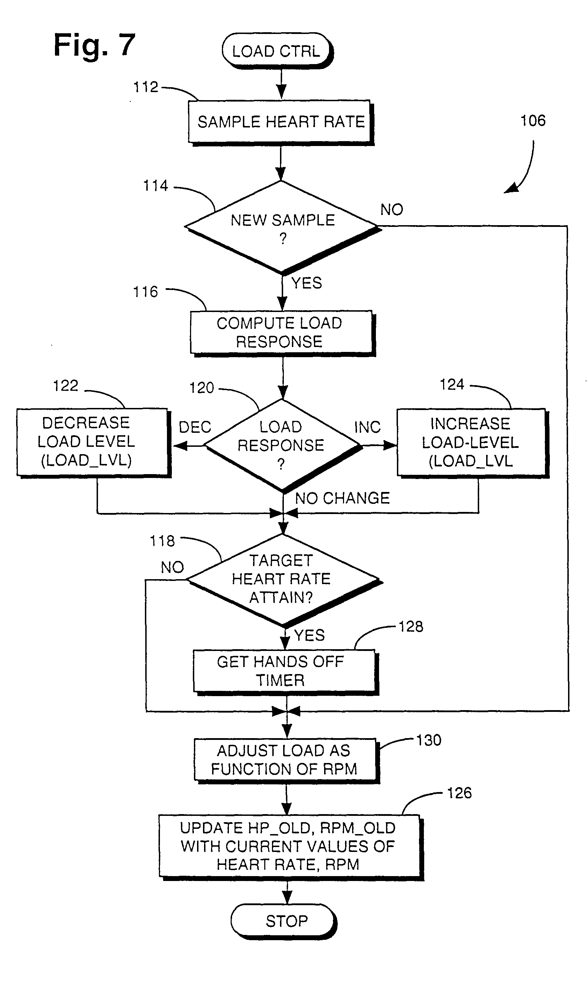

FIG. 7 is a logic flow chart of the load control module 106. At a block 112, microcomputer 54 interrogates heart rate monitor 36 to determine if a current sample of the user's heart rate is available. If such a sample is available, it is stored in a variable HR_NEW.

At a decisional block 114, if a new sample was acquired at block 112, then control continues to a block 116, where the change in load resistance (the "load response") is computed on the basis of the freshly sampled heart rate and other variables as discussed below. Otherwise control skips the block 116 and jumps to a block 130, discussed below. After execution of the block 116, control branches at a decisional block 120 depending on whether the load response is positive or negative. If load response is negative, then the microcomputer 54 decreases load resistance by calling a decrease load routine shown in a block 122. If load response is positive, then the microcomputer 54 increases load resistance by calling an increase load routine shown in a block 124.

After load resistance is adjusted, control moves to a block 118, where microcomputer 54 determines whether the user has attained the target heart rate (the "target heart rate condition"). Preferably, a boolean flag (TARGET) indicates whether the target heart rate condition exists or has existed. The TARGET flag is set at the block 112 as soon as the user's current heart rate is greater than or equal to the target heart rate. Once the target heart rate condition is achieved, the TARGET flag remains set for the duration of the exercise, even if the user's actual heart rate subsequently falls below the target heart rate.

If the user has not yet reached the target heart rate condition, then control skips to the block 130, discussed below. If the user, however, has attained the target heart rate, then the microcomputer 54 calls a set-hands-off-timer routine shown at block 128 and discussed below in greater detail. The hands-off-timer routine examines the change in the user's heart rate over time, and on the basis of that examination, sets a time period during which the user is free to remove his hands from sensors 38.

After the hands-off timer is set, microcomputer 54 adjusts load resistance as a function of the change in the user's rpm, as illustrated by the block 130. As discussed above, the idea behind adjusting load resistance as a function of RPM is to take advantage of the information about the user's exercise intensity that can be gleaned from rpm.

As the user pedals faster or slower at a particular level of load resistance, the user's heart rate will fluctuate. The change in the rate of rate of exercise is an externally observable physical indicator of exercise intensity, and is in fact a predictive measure of future changes in the user's true physiological exercise intensity, as measured by heart rate, for example. By increasing the load when rpm decreases (and, conversely, decreasing the load when the rpm increases), microcomputer 54 can reduce fluctuations in user heart rate which would otherwise result from the change in pedal rpm.

By using rpm as an indicator of exercise intensity, system 52 can maintain the user's heart rate even when heart rate data is unavailable, such as when the hands-off timer is set. This enables the system 52 to set the hands-off timer (discussed above) for relatively long periods such as ninety seconds. Moreover, the user is free to change the rate of pedaling to make exercise more interesting.

After setting the hands-off timer and adjusting load resistance as a function of rpm, the microcomputer 54 performs housekeeping functions illustrated by the block 126. Specifically, the current values of rpm and heart rate (RPM_NEW and HR_NEW) are preserved in memory (in RPM_OLD and HR_OLD, respectively) so that the changes in rpm and heart rate may be determined during the next iteration of the load control module 106.

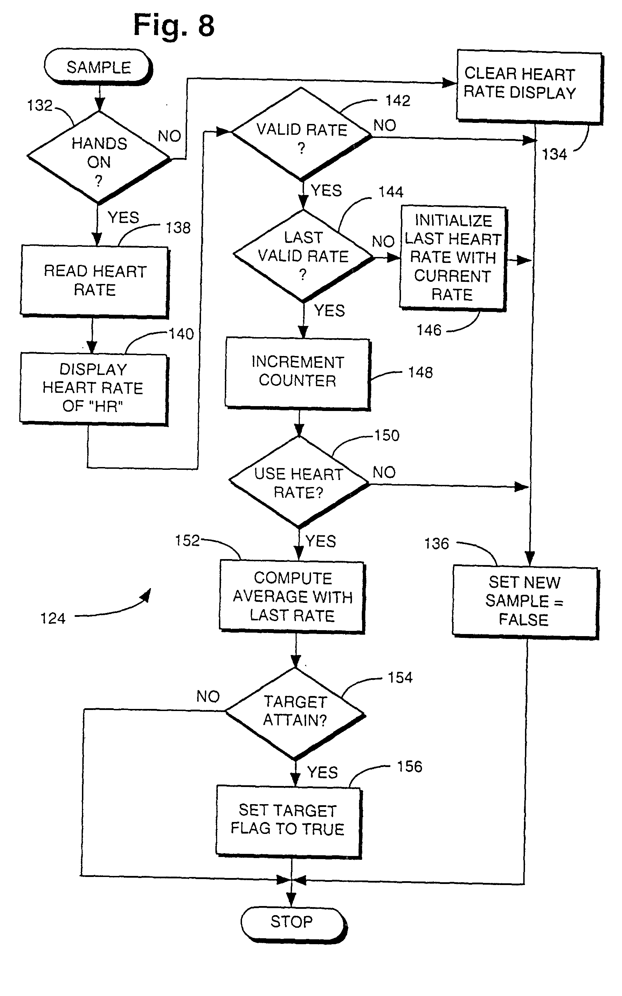

The functions of the load control module 106 discussed above are now considered in greater detail. Referring to FIG. 8, the sample-heart-rate routine 112 is more fully described. In accordance with this routine, the microcomputer 54 periodically samples the output of the heart rate monitor 36. It will be noted that the specific techniques disclosed in connection with sample routine 112 have been found to work well, but represent just one of many alternatives which will be readily apparent to those skilled in the art.

Beginning at a block 132, the condition of the equipment signal or "hands-on" bit (provided by the heart rate monitor 36 at the line 72) is determined. If the user is not engaging the sensors 36, it means that no current heart rate data is available. As shown at a block 134, the microcomputer 54 clears the LED heart rate display 74 and disables the LED hands-on indicator 78 of the control panel 30 (as shown in FIG. 5). The microcomputer 54 then sets the new-sample flag (used in connection with the block 114 discussed above) to false, as shown in a block 136.

If, however, the user is engaging the pulse sensors 38 (the "hands-on condition"), then the microcomputer 54 reads the user's heart rate (provided by the heart rate monitor 36 at the line 70), as shown at a block 138. This heart rate is then stored in a memory variable (HR_NEW) and displayed on the LED heart rate display 74. The LED hands-on indicator 78 is also enabled. Thus, each time the user places his hands on the pulse sensors 38, his heart rate is displayed on control panel 30.

If, as may be the case initially, the user's hands are on the pulse sensors 38 but heart rate monitor 36 has not yet generated a valid heart rate reading (i.e. a value of between 50 and 200 beats per minute), then the microcomputer 54 generates an "Hr" or other suitable symbol in the LED heart rate display 74.

At a decisional block 142, if the newly sampled heart rate is not valid, then microcomputer 54 skips to the block 136 where the new sample flag is set to false. As discussed below, the heart rate value used to adjust load is preferably an average of the current and last in-assured heart rates (HR_NEW, HR_OLD). Thus, if the current heart rate sample is valid, the microcomputer 54 proceeds to a decisional block 144 where it examines the last measured heart rate (HR_OLD) stored in the memory 58. If the last measured heart rate is invalid, the microcomputer 54 skips to a block 146,
where the value of HR-OLD is updated with the value of HR-NEW. Control then moves to the block 136, where the new sample flag is set to false.

If the last measured heart rate is valid, the microcomputer 54 continues to a block 148, where it increments a counter. The function of the counter is to track iterations of the sample heart rate routine 112. Depending on the performance of heart rate monitor 36, it may be desirable to use every other heart rate sample. In this case, if the value of the counter is an even number, then at a block 150 the microcomputer 54 uses the currently measured heart rate to compute an average heart rate, as illustrated at a block 152. Otherwise, the currently measured sample is discarded, and control skips to the block 136, where the new sample flag is set to false.

Another use of the counter is to suppress the very first sample of heart rate data after the user has placed his hands on the sensors 38. This can be accomplished by setting the counter to -1 at the block 146. In this manner, once heart rate monitor 36 begins delivering valid heart rate data to the microcomputer 54, the first valid sample (corresponding to a counter value of -1) is discarded.

The average heart rate computed at the block 152 is computed by taking the average of the old and current heart rate values (i.e. (HR_OLD plus HR_NEW)/2), and storing the result as the current heart rate. By computing this running average, minor aberrations and fluctuations in heart rate data are filtered to improve system stability.

The microcomputer then compares the average computed heart rate (HR_NEW) to the target heart rate (HR_TGT) stored in the memory 58, as shown at a block 154. If the average computed heart rate (HR_NEW) is greater than or equal to the target heart rate, a flag (TARGET) is set true, as illustrated in a block 156. The TARGET flag remains true for the duration of the exercise period, and indicates that the user has reached target heart rate condition.

Upon completion of the blocks 156 or 136, as the case may be, the sample-heart-rate routine 124 is terminated, and control returns to the load control module 106 illustrated in FIG. 7. As discussed above, if the sample-heart-rate-routine has successfully acquired a sample heart rate, the new sample flag will be set to true, and, as illustrated in the block 114 of FIG. 7, the microcomputer 54 will proceed to compute a change in the load signal or "load response", as illustrated in the block 116.

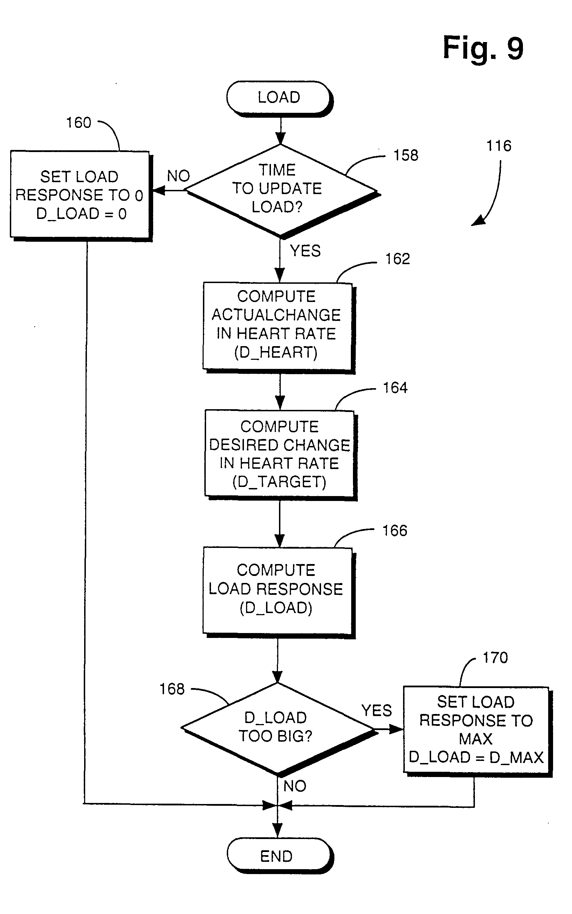

Referring to FIG. 9, the operation of the load response routine 116 is illustrated in greater detail.

The function of the load response routine 116 is to determine a load response, i.e. the amount by which microprocessor 54 changes the value of the load signal 66 to maintain or attain the target heart rate.

The microprocessor 54 first determines at a block 158 whether it is time to update the load signal. While load response can be updated with each iteration of the load response routine, it may be preferable to update less frequently to avoid excessive changes in load resistance. To this end, load response can be updated on each n^{th} iteration, where n is a number such as 6. This can be determined by examining the incremented value of the counter (HR_CTR) at the block 148 to determine if the counter's current value is a whole number multiple of n.

If it is not time to update the load signal 66, the microcomputer 54 sets the load response variable (D_LOAD) to zero, as illustrated at a block 160, and terminates the load response routine 116. If it is time to update the load signal 66, then microcomputer 54 calculates the load response (D_LOAD) as illustrated by blocks 162 through 170 of FIG. 9. First, the actual change (D_HEART) in the user's heart rate is computed by subtracting the current heart rate value (HR_NEW) from the old heart rate value (HR_OLD). Next, the desired change in heart rate (D_TARGET) is computed by subtracting the target heart rate value (HR_TGT) from the current heart rate value (HR_NEW). Finally, the load response (D_LOAD) is calculated as the difference between the desired change (D_TARGET) in heart rate minus the actual change (D_HEART) in heart rate multiplied by a scaling constant (K₁) (i.e.(D_TARGET - D_HEART) * K₁).

The scaling constant K₁ is determined by empirical calibration of the particular device in which the invention is implemented, and simply serves to convert the calculated difference between D_TARGET and D_HEART into units of load resistance. In some cases, performance may be improved by weighting D_TARGET relative to D_HEART.

It will be noted that the foregoing technique computes load response as function of the differential in heart rate. Other techniques for calculating load response as a function of heart rate are known, and the foregoing is offered as one which we have found particularly effective. However, the invention contemplates the use of any suitable technique. In accordance with one alternative, for example, the load response could be computed (albeit less effectively) as a function of the difference between actual and target heart rate.

As a precaution, the calculated load response is compared to a predetermined maximum (D_MAX), as shown in a block 168. If the load response (D_LOAD) exceeds the predetermined maximum, then load response is set to the predetermined maximum. It will be noted that load response represents the change in load, as opposed to the total magnitude of load. Thus, the predetermined maximum (D_MAX) does not represent the maximum load to which a user may be subjected, but rather the maximum increase which may be imposed during one iteration of the load control module 106. This prevents load from increasing at an excessive rate which might otherwise prematurely exhaust the user.

Referring back to FIG. 7, after load response (D_LOAD) is computed at the block 116, control branches at the decisional block 120 depending on whether load response is positive, negative or zero. If the load response is negative, then the load signal is adjusted to decrease total load resistance, as shown in the block 122. If the load response is positive, then the load signal is adjusted to increase the total load resistance, as shown in the block 124. If the load response is zero, no change in load resistance is required, and control continues to the block 118.

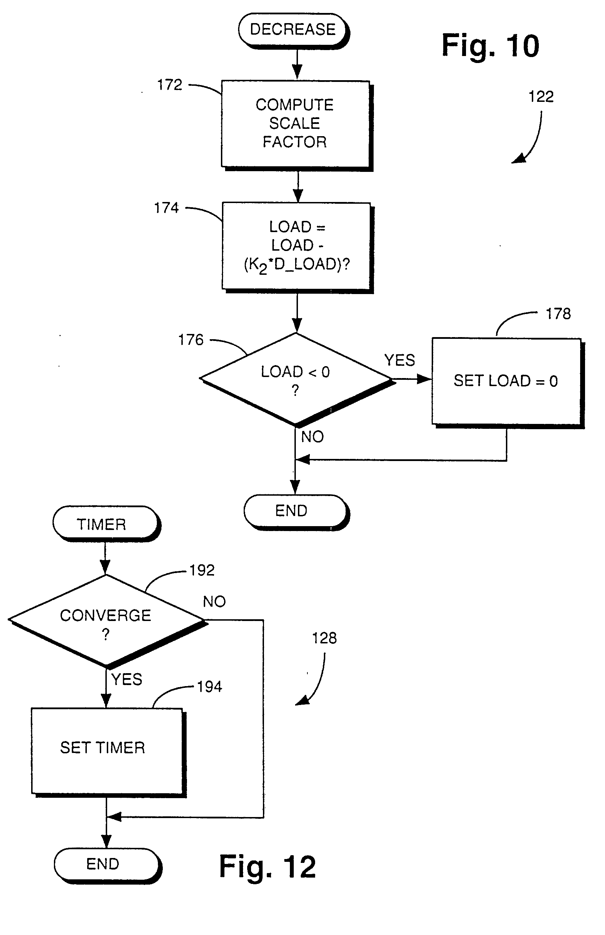

Referring to FIG. 10, the decrease load routine shown at the block 122 of FIG. 7 is illustrated in greater detail. Before adjusting the load signal, microcomputer 54 calculates a scaling factor K₂, which may simply be a constant such as 1.25. The scaling factor K₂ is determined by empirical calibration of the particular device in which the invention is implemented, and simply serves to convert the calculated load response (D_LOAD) into the desired units of load resistance.

Scaling factor K₂ may also be a function of the user-entered load level (LEVEL) . It is the case that users who enter high initial load levels typically require more dramatic adjustments to level so as not to overshoot the target heart rate. To compensate for this phenomenon, the scaling factor K₂ may be computed in accordance with the following formula:${\text{K}}_{\text{2}} \text{= 1.25 + LEVEL/6.9}$

In practice, specific embodiments should be calibrated and empirically determined values may be used in lieu of 1.25 and 6.9 above.

After the scaling factor K₂ is calculated, the load signal is adjusted as shown by block 174 of FIG. 10. Specifically, the value of load in memory 58 (LOAD) is arithmetically decreased by a value equal to the scaling factor multiplied by the load response (LOAD = LOAD - (K₂ * D_LOAD). If the resulting value of load resistance is less than zero, then load resistance is set equal to zero, as shown in blocks 176 and 178.

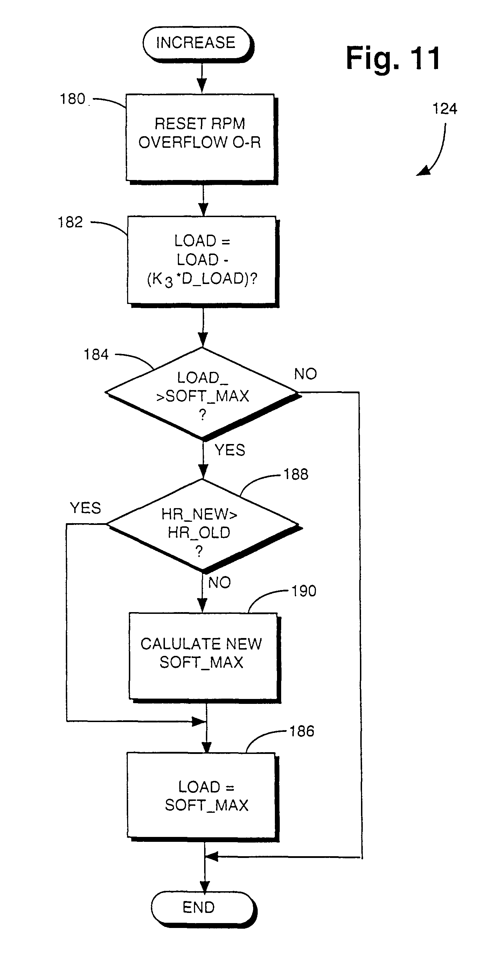

Referring to FIG. 11, the increase load routine shown at the block 124 of FIG. 7 is illustrated in greater detail. Before adjusting the load signal, microcomputer 54 calculates a scaling factor K₃. The scaling factor K₃ is determined by empirical calibration of the particular device in which the invention is implemented, and simply serves to convert the calculated load response (D_LOAD) into the desired units of load resistance. Scaling factor K₃ may also be set to arbitrarily reduce the load increase. For example, even if the load response does not require scaling, it may be desirable for safety and comfort considerations to use a value of 0.75 for K₃ to reduce the increase load response.

Microcomputer 54 may also reset an rpm overflow counter (RPM_CTR), as shown in a block 180. The function of the rpm overflow counter is discussed below in more detail.

After the scaling factor K₃ is calculated, the load signal is adjusted as shown by block 182 of FIG. 11. Specifically, the value of load resistance in memory 58 (LOAD) is arithmetically increased by a value equal to the scaling factor K₃ multiplied by the load response (LOAD = LOAD + (K₃ * D_LOAD). If the resulting value of load resistance (LOAD) does not exceed a predetermined soft maximum value (SOFT_MAX), then the increase load routine 124 terminates.

If the resulting value of load resistance (LOAD) does exceed the soft maximum (SOFT_MAX), then load resistance may be set equal to the soft maximum, as shown in a block 186. The soft maximum is preferably calculated at the beginning of exercise as a function of the user-selected exercise level (LEVEL), which is entered at block 98 of FIG. 6. It will be noted that the difficulty of exercise level (LEVEL) selected by the user is typically indicative of the user's overall fitness level (or at least the user's perceived fitness). Thus, the value of the soft maximum can be higher where the user's has selected a more difficult initial exercise level (LEVEL).

For example, if the load resistance has a range of 0 through 255 (corresponding to possible numerical values of the load signal), and the user selects an initial load level (LEVEL) of 6 (out of 12), then the user-selected load level could be said to correspond to a load signal of 125 (about one-half of 250). Preferably, the soft maximum (SOFT_MAX) would be set to 127.

As illustrated at a block 188 of FIG. 11, it is possible that a user's heart rate is decreasing (i.e. HR_NEW < HR_OLD) even though load resistance has reached the soft maximum. In this case, it is desirable to gradually increase the soft maximum, as shown by a block 190. Thus, at each iteration of the block 190, the value of the soft maximum is incremented, such as by one, until the value of the soft maximum reaches a predetermined hard maximum (HARD_MAX).

The value of the hard maximum is determined in accordance with the following formula:$\text{HARD_MAX = SOFT_MAX + (0.25 * (MAX - SOFT_MAX),}$ where SOFT_MAX is equal to the initial value of the soft maximum (i.e. user-selected level), and MAX is equal to largest value of load resistance which load device 62 can impose on the user.
By adjusting load resistance in accordance with the user's heart rate, such as described above, the system 52 will tend to establish and maintain the user's heart rate at or near the target heart rate. Preferably, during steady state exercise, the user's heart rate should be within 5 beats per minute of the target heart rate. We have found that even better results have been obtainable with devices built in accordance with this invention.

Referring back to FIG. 7, after the load signal has been adjusted at blocks 122 and 124, as the case may be, the microcomputer 54 determines whether a target heart rate condition exists, as shown by block 118. Before a user reaches his target heart rate, it is necessary to closely monitor the user's heart rate so that the appropriate load value can be selected. Once the user reaches his target heart rate, it is no longer necessary to continually monitor heart rate. This is significant because the user may wish to remove his hands from sensors 38.

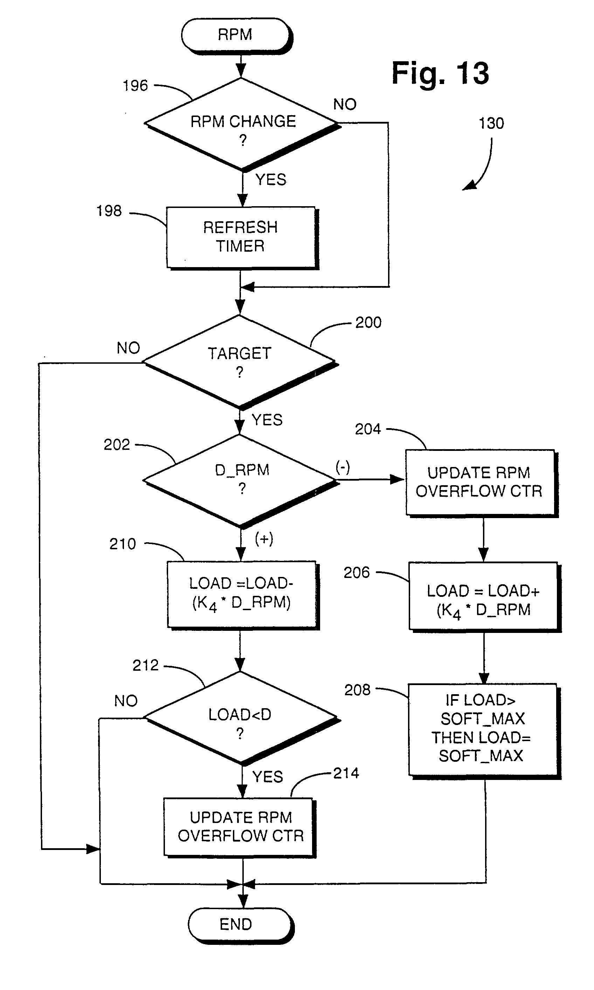

Thus, if the user has reached a target heart rate, the microcomputer 54 will determine if it is possible for the user to remove his hands from the pulse sensors 38, and if so, for how long. This determination is performed by the set-hands-off-timer routine shown at the block 128. The details of the set-hands-off-timer routine are illustrated in FIG. 12. The microcomputer 54 calculates the difference between the actual and desired rates of change in heart rate (D_TARGET - D_HEART), as shown in block 192. If the absolute value of the difference is below a predetermined threshold (preferably 8), then control continues to a block 194. Otherwise, the routine terminates. At the block 194, a time limit is selected in accordance with the following hands-off time limit table:

| Time Limit (sec) | Difference |
|---|---|
| 15 | 6-7 |
| 30 | 4-5 |
| 60 | 2-3 |
| 90 | 0-1 |

Preferably, the time limit is set to 15 seconds for the first iteration of the block 194 regardless of the actual difference between D_HEART and D_TARGET.

The selected time limit is used to set the a hands-off timer, which may be a memory location that is periodically decremented by an interrupt generated by the timer 60. Thus, the contents of the hands-off timer (HR_TMR) at any given moment represent the period of time in seconds remaining in which the user need not engage pulse sensors 38. The value contained in the hands-off timer is automatically decremented by one each second. Preferably, the hands-off timer is reset to zero if microcomputer 54 detects a hand-on condition at the block 132.

It is understood that the present invention is by no means limited to heart rate detectors in which the user places his hands on electrodes. The foregoing routines are readily adaptable to any type of system wherein the user somehow engages a pulse sensor. For example, in a conventional ear-clip type detector, the hands-on signal described above would be replaced by a signal indicating whether the detector was engaged with the user's ear. When the hands-off timer has a positive value, the user would be permitted to remove the ear clip.

Referring back to FIG. 7, after the hands-off timer has been set, the microcomputer 54 proceeds to block 130, where the load resistance is adjusted as a function changes in pedal rpm or other measure of the rate of exercise. It will be noted that pedal rpm is an externally observable physical indicator of the user's exercise intensity (as opposed to a physiological indicator such as heart rate). It will be noted that the rpm-based adjustment at block 130 takes place even if no new heart rate data is available (i.e. the new sample condition is false). In this manner, the invention provides for heart rate maintenance even during times when heart rate data is not available.

The operation of the rpm-based adjustment routine 130 is illustrated in greater detail by FIG. 13. As shown in blocks 196 and 198, microcomputer 54 initially reads the current value of rpm from tachometer 64. The current value (RPM_NEW) is compared to the last value (RPM_OLD). If the change in rpm (D_RPM = RPM_NEW - RPM_OLD) is greater than a predetermined threshold (such as 6 rpm), then the hands-off timer is reset to the minimum time period (in this case, 15 seconds) set forth in the hands off time limit table discussed above, unless the hands-off timer already has a value below that minimum time. The rationale behind resetting the hands-off timer is that great fluctuations in pedal rpm are likely to be accompanied by great fluctuations in heart rate, and, therefore, additional heart rate data is required.

The microcomputer 54 then determines whether a target heart rate condition has been attained, as shown in the block 200. If the condition has not been attained, the rpm-based adjustment routine 130 terminates without further action. No rpm-based adjustment is required in this case because until the target heart rate has been obtained, the user maintains his hands on the sensors 38, providing a steady stream of heart rate data which can be used by the load response routine 116 to control heart rate.

As shown at a block 202 - 206, if rpm is decreasing (D_RPM <0), then the load resistance is adjusted upward by arithmetically adding the change in rpm (multiplied by a scaling constant, K₄, if necessary) to the value of load resistance stored in memory. Thus,${\text{LOAD = LOAD + (K}}_{\text{4}} \text{* D_RPM)}$

It will be noted that before the change in rpm is added to the value of load, it is first subtracted from the value of the rpm overflow counter (RPM_CTR), as shown in the block 204. The rpm overflow counter is explained below in detail.

At the block 204, the rpm overflow counter is reduced to a value no lower than zero by the amount of the change in rpm (D_RPM). If the change in rpm is greater than or equal to the value of the rpm overflow counter, then the change in rpm is reduced by the value of the rpm overflow counter,$\text{D_RPM = D_RPM - RPM_CTR}$ and the rpm overflow counter is set to zero. Otherwise, the value of the rpm counter is reduced by the change in rpm,$\text{RPM_CTR = RPM_CTR - D_RPM,}$ and the change in rpm is set to zero.

After the value of load resistance has been adjusted by the change in rpm, the resulting value of load resistance (LOAD) is compared to the current value of the maximum (SOFT_MAX), as shown in a block 208. If the resulting value of load resistance exceeds the soft maximum, then load resistance is set equal to the soft maximum.

Alternatively, if at the block 202 rpm 15 increasing, then control continues to a block 210 where the load resistance is adjusted downward by arithmetically subtracting the change in rpm (multiplied by a scaling constant, K₅, if necessary) to the value of load resistance stored in memory. Thus,${\text{LOAD = LOAD - (K}}_{\text{5}} \text{* D_RPM)}$

As shown in blocks 210 and 214, if the resulting value of load resistance is less then zero (i.e. LOAD < 0), then the load resistance is set to zero, and the rpm overflow counter is incremented by the amount by which the term K₅ * D_RPM exceeds the initial value of load.

It will be observed that, because load resistance may not take a negative value, the rpm overflow counter preserves the amount by which load resistance would otherwise be negative. As discussed above, when microcomputer 54 adds to load resistance on the basis of decreasing rpm (such as at the blocks 204-206), it first attempts to deplete the overflow represented by the rpm overflow counter, as discussed above. For example, suppose a user accelerates pedaling (i.e. increases rpm) over a period of time, and, at some point during this acceleration, load resistance is driven to zero by repeated iterations of the blocks 210 and 214.

Because the rpm overflow counter will contain a non-zero value, the load resistance will not immediately be increased when the user's acceleration peaks and begins to decline. Rather, the load resistance will remain at zero, while the rpm overflow counter absorbs the changes in rpm. When the value of the rpm overflow counter is reduced to zero, successive reductions in pedal rpm will begin to result in higher load resistance.

### 3. Display Module

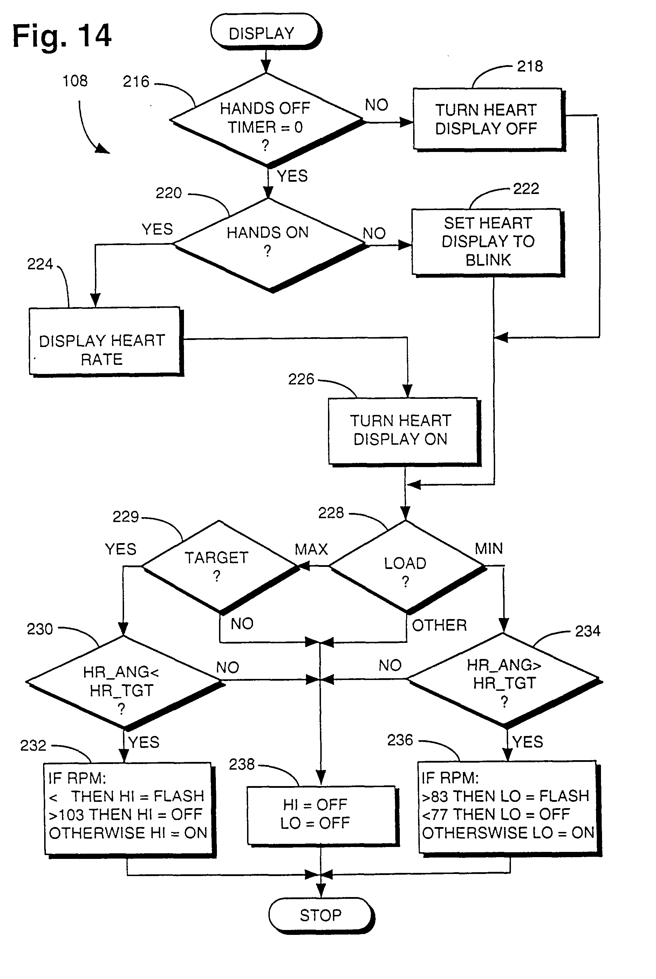

Referring back to FIG. 6, it will be noted that the display module 108 is called with each iteration of the main driver loop 102. The display module manages the display of information to the user via the control panel 30. Referring to FIG. 14, the operation of the display module 108 is illustrated in greater detail. The primary function of the display module 108 is to prompt the user when to place his hands on the pulse sensors 38, and to advice the user when he is free to remove his hands.

As shown in blocks 216 - 222, the microcomputer 54 initially determines whether the hands-off timer has a value equal to zero (HR_TMR = 0). It will be recalled that the hands-off timer is set by the load control module 106 (discussed above in connection with FIG. 12) to specify periods when the user is free to remove his hands from the sensors 38. Thus, when the hands-off timer is at zero, the hands-off period is over.

The system 52 requires additional heart rate data, and it is time for the user to place his hands back on the sensors 38. If the hands-off timer is not at zero, then the system 52 does not yet require current heart rate data. Microcomputer 54 turns off the LED heart screen, as shown in the block 218, thereby advising the user that he is free to remove his hands from the sensors.

If the hands-off time is equal to zero, the microcomputer determines if a hands-on condition exists, as shown at the block 220. If the user's hands are not on the sensors 38, microcomputer 54 sets the LED heart screen 76 to blink, as shown at the block 222. The microcomputer 54 also disables the heart-shaped LED hands-on indicator 78 and clears the LED heart rate display 74. This prompts the user to place his hands on the pulse sensors 38. If, however, the user's hands are already on the sensors 38, then the microcomputer enables the LED hands-on indicator 78, displays the current value of heart rate (HR_NEW) or the "Hr" symbol on the LED heart rate display 74, and turns on the LED heart screen. It may also be desirable to display the user's heart rate whenever the user touches the pulse sensors, regardless of whether the hands-off timer is zero.

As shown in blocks 228-236, the display module also examines the value of load resistance (LOAD), and determines whether load is at the value of the soft maximum (SOFT_MAX), or at the value of zero, as shown at the block 228. If the load resistance is equal to the hard maximum, then the TARGET flag is examined at the block 229 to determine if the user has ever reached the target heart rate. If TARGET is false, then the display module terminates. Otherwise, if TARGET is true, then current heart rate is examined to determine if it is less than the target heart rate, as shown in the block 230. If the user has reached the target heart rate, the display module terminates. Otherwise, the current value of rpm is examined, as shown by the block 230. As illustrated at the block 232, if the user's rpm is below 103, then the high rpm LED 80 is enabled. This prompts the user to maintain a pedal rpm near the high level of 100 (indicated by the indicia 92 on panel 30, as shown in FIG. 5). If the user's rpm level is below 98, then the high rpm LED is set to blink so as to prompt the user to increase pedal rpm to 100.

Contrastingly, if the load resistance is at a minimum, and the user's heart rate is at or below the target heart rate, then the display module terminates, as shown by the block 234. However, if the user's heart rate is above the target heart rate, then the current value of rpm is examined, as shown by the block 234. As illustrated at the block 236, if the user's rpm is above 82 then the low rpm LED 82 is set to blink. This prompts the user to reduce pedal rpm to the low rpm level of 80 (indicated by the indicia 94 on panel 30, as shown in FIG. 5). If the user's rpm level is between 77 and 82, the low rpm LED 82 is turned on. This prompts the user to maintain pedal rpm near 80 rpm.

If the user's rpm is below 77, the low rpm LED 82 is turned off.

### 4. Safety Module.

Referring back to FIG. 6, it will be noted that the safety module 110 is called with each iteration of main driver loop 102. The function of the safety module is to ensure that the system 52 does not continue to operate without sufficient heart rate data. Unlike some existing systems, the safety features of the present invention do not disable the bicycle 20 or even warn the user when the user's heart rate reaches a critical level. Rather, the safety features of the present invention take effect when there is an absence of new heart rate data for a prolonged period of time.

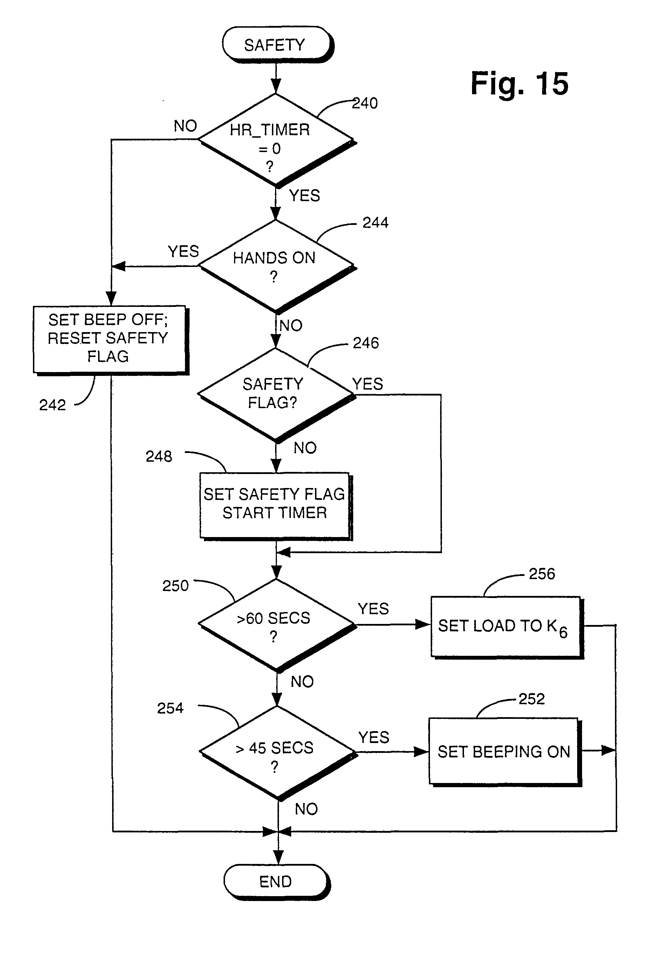

The operation of the safety module 110 is illustrated in greater detail by FIG. 15. As shown in a block 240, the microcomputer 54 determines whether the hands-off timer (HR_TMR) is currently set to zero. If not, then it is permissible for the user to have his hands off of the sensors 38, and no further action is necessary. The safety module disables the bell 85, as shown at a block 242, and terminates.

If, however, the hands-off timer is equal to zero, then the hands-on signal is examined, as shown in a block 244. If the hands-on signal indicates a hands-on condition, then the safety module disables the bell 85, as shown at block 242, and terminates.

If instead the hands-off timer is zero and the user's hands are off, the safety module sets a safety flag and starts a safety timer, as shown in blocks 246 and 248. The safety timer may be implemented using timer 60 of microcomputer 54 in any suitable manner, and in practice is a counter that is periodically incremented by a timer interrupt. The safety timer should not be confused with the hands-off timer.

During this and each subsequent iteration of the safety module 110, the value of the safety timer is examined, as shown in blocks 250-256. When the timer reaches 45 seconds, the microcomputer 54 enables the bell 85, as shown in the block 252. The beeping sound augments the flashing LED heart screen (set by the display module 108) to advise the user to place his hands on the pulse sensors 38. If the user does not place his hands on the pulse sensors 38, the safety flag will remain on, and the timer will continue to increment. If the timer reaches sixty seconds, the load resistance is set to a predetermined value K₆, which may be zero or other low value of load, as shown in the block 256, to provide the user with additional incentive to place his hands on the sensors 38, and to reduce the risk that the user may attain an excessive heart rate.

It will be noted that when the user places his hands on the sensors 38, the safety flag is reset on the next iteration of the safety module, as shown in the block 242.

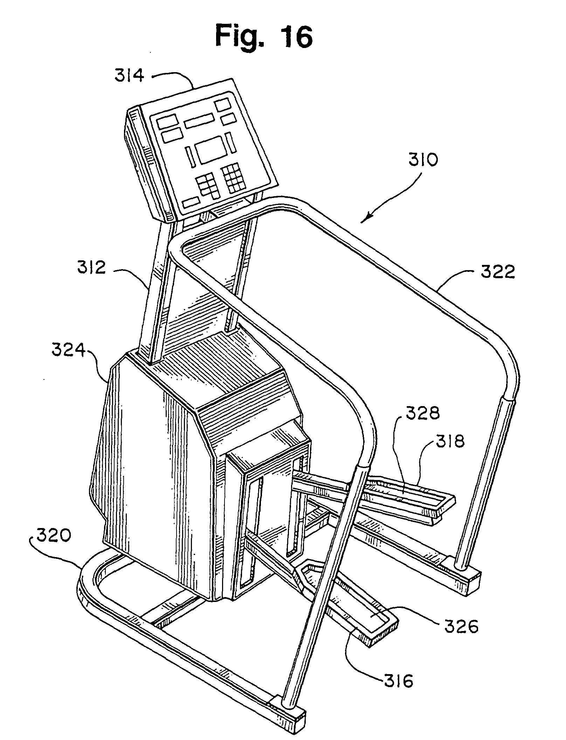

As to a preferred aspect of the invention, FIG. 16 shows a climbing-type exercise apparatus 310 that includes a frame 312, a control panel 314, and a pair of pedals 316 and 318. The frame 312 may be of any suitable construction. In the illustrated embodiment, the frame 312 includes a base 320, a tubular section 322, which can be used as a handrail, and a housing 324. The pedals 316 and 318 each include pads 326 and 328, respectively, which form the tread portion of a simulated staircase or hill.

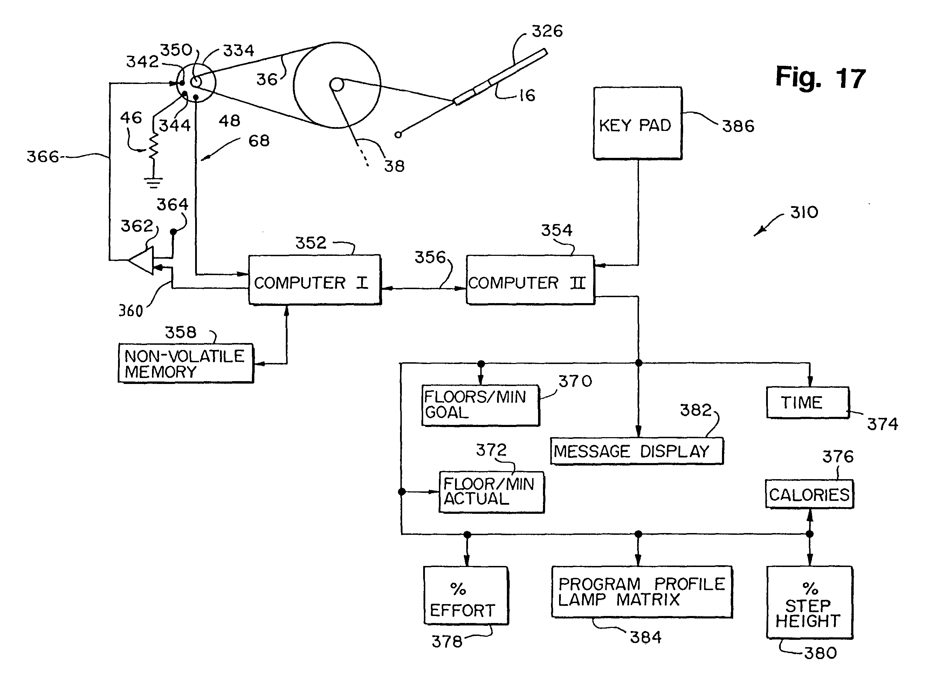

Pedals 316 and 318 are pivotally mounted to the base 320 to allow the user to vertically reciprocate pads 326 and 328 in an exercise motion. As best seen in FIG. 17, a suitable transmission device 330 is disposed in the housing 324 and converts the vertical reciprocating motion of the pedals 316 and 318 into a rotary motion of a flywheel 332. The rotary motion of the flywheel 332 is resisted by an alternator 334, which is coupled to the flywheel 332 by a suitable belt 336.

Transmission device includes a length of the chain 338, which cross connects the pedals 316 and 318. Only a portion of the chain 338 is illustrated in FIG. 17. The chain 338 drivingly engages a pair of sprockets 340 which are coaxially mounted on opposing sides of the flywheel 332. For clarity, only one of the sprockets 340 is illustrated in FIG. 17. One-way clutches (not illustrated) are coupled to each of the sprockets 340 so that the flywheel 332 rotates in only one direction, notwithstanding the fact that the sprockets 340 will rotate bidirectionally due to the reciprocating motion of the chain 338. The invention may be practiced with other suitable transmission devices.

The alternator 334 is a commercially available alternator such as commonly used in automotive applications, and includes a field coil terminal 342, a driven coil terminal 344 that is connected to ground via a 0.5 ohm load resistor 346, and a tap terminal 348. The alternator 334 also includes a rotatable shaft 350 which is driven by the belt 336. As the user vertically reciprocates pedals 316 and 318, he or she drives flywheel 332, which in turn drives alternator shaft 350. The rotation of shaft 350 causes alternator 334 to generate a voltage potential at driven coil terminal 344. The resulting current flows through the resistor 346, thereby dissipating the user's kinetic energy in the form of heat. The resistance force or "load" in opposition to the rotation of shaft 342 (and, consequently, to the user's reciprocating exercise movement) is governed by the current flowing through field coil terminal 342.

The operation of exercise device 310 is controlled by a pair of computers 352 and 354, which are coupled together via a serial communication link 356. Each of computers 352 and 354 includes dynamic memory and input-output interface circuitry, and computer 348 also includes non-volatile memory 358. Computer 352 controls the load resistance of alternator 334, and is preferably a Motorola™ 68HC05 microcontroller. Computer 352 also calculates the rate of user exercise, the calories consumed, and other exercise related data. Computer 354 manages user input and output via panel 314, and is preferably a Motorola™ 68HC05 microcontroller. Alternatively, a single, integrated computer can be used.

Computer 352 generates as output a control signal 360, which is amplified by an amplifier 362. The output 364 of amplifier 362 is coupled to the field coil terminal 342, and the input of amplifier 362 not connected to control signal 360 is tied to a fixed voltage source 366.

Control signal 360 is a pulse train. The pulses are generated at regular intervals of approximately 350 milliseconds. The duration (or "duty cycle") of each pulse, however, is varied by computer 352 to modulate the effective current level through field coil terminal 342.

It will be seen that computer 352 can control the load resistance by modulating the duty cycle of the control signal 360. Specifically, a greater duty cycle increases the effective current through field current terminal 342, and causes alternator 334 to impart greater load resistance in opposition to the rotation of shaft 340 (and, consequently, to the user's reciprocating exercise movement). In the preferred embodiment, the duty cycle does not exceed fifty percent.
An input 368 of computer 352 is coupled to the tap terminal 348 of alternator 334. As the alternator shaft 350 rotates, a sinusoidal signal emanates from tap terminal 348. The peak portions of that signal strobe input 368, generating a hardware interrupt at a frequency equal to the rate of rotation of shaft 350. The computer 352 can time the period between these interrupt signals to calculate the rate of the user's exercise movement.

### Control Panel

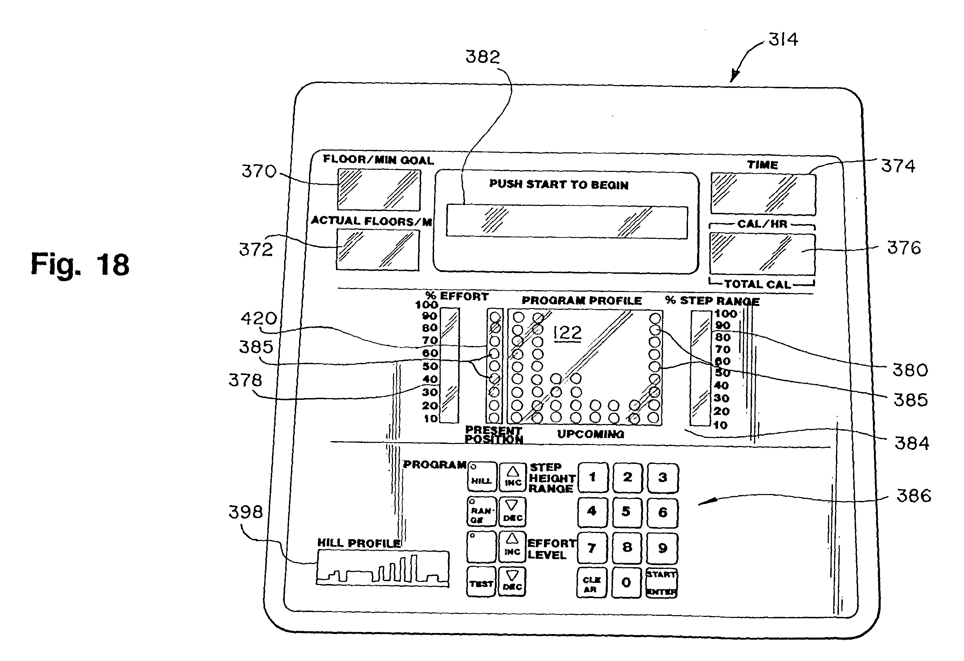

Referring now to FIGS. 17 and 18, it will be seen that computer 354 is linked to the control panel 314 for managing user input-output. The panel 314 includes displays designed to accommodate operation of exercise device 310 in a "regular" and a "climbing" mode, although the invention may be practiced without the regular mode. In the regular mode, the exercise device 310 operates as a conventional system, wherein the user's effort level is controlled by regulating the rate at which the user exercises. To this end, panel 314 includes a display 370 for prompting the user to exercise at a particular rate (expressed in floors per minute), and a display 372 of the user's actual rate of exercise.

The panel 314 includes informational displays 374 and 376 of elapsed time and expended calories, respectively, as well as displays 378 and 380 of percentage of effort and step height, respectively. An alphanumeric display 382 displays messages to the user including instructions on how to use device 310.

The display 378 is a column of LED's, where the individual LEDs are lit in sequence to indicate to the user the percentage of effort that he or she is expending. In this case, the percentage of effort is the percent the actual rate of exercise bears to the target rate.

The display 380 is also a column of successively lit LEDs which graphically illustrates the percentage which the user's actual displacement of pedal members 316 and 318 bears to a target displacement. The actual displacement may be measured by a position sensor (not shown).

In the climbing mode, the exercise device 310 operates in accordance with the invention. Displays 370, 378 and 380 need not be used because in the climbing mode exercise device 310 does not regulate the user's rate of exercise. Rather, exercise device 310 regulates (independent of exercise rate) the resistance which alternator 334 imparts to the user's reciprocating exercise motion. As discussed below in greater detail, the current and upcoming levels of resistance are displayed in a program profile lamp matrix 384. Matrix 384 includes an array of LEDs 385. For clarity, not every one of LEDs 385 is illustrated in FIG. 18. Alternatively, other sources of illumination such as LCD, for example, may be used.
Panel 314 also includes a keypad 386 which enables the user to select programs, modes, and enter data such as weight. As illustrated in FIG. 18, the keypad 386 includes keys A and B for selecting one of the above-described regular and climbing modes, respectively. Keys H, M and R enable the user to select a program for operation in the selected one of climbing and regular modes. The available exercise programs are Hill, Manual and Random, and are selected by pressing the H, M and R keys, respectively. These programs are described below with respect to the climbing mode.

Periodic update of the information displayed on the panel 314 is performed by the computer 354 using information provided by the computer 352 and transmitted to the computer 352 via the link 356. Keypad 386 is monitored by the computer 354 and, the user's input is transmitted to the computer 352 via the link 356.

### Climbing Mode

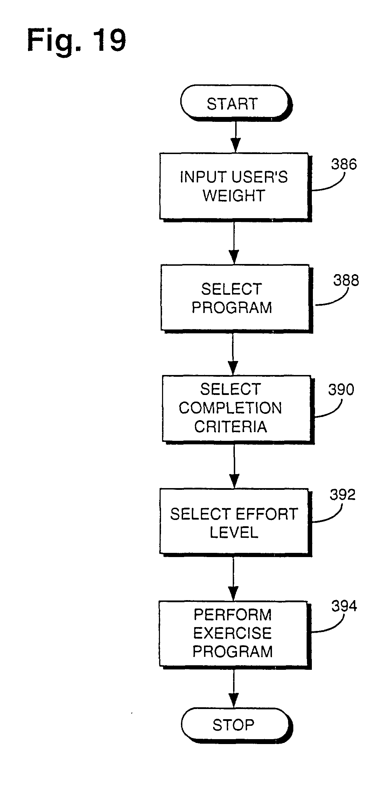

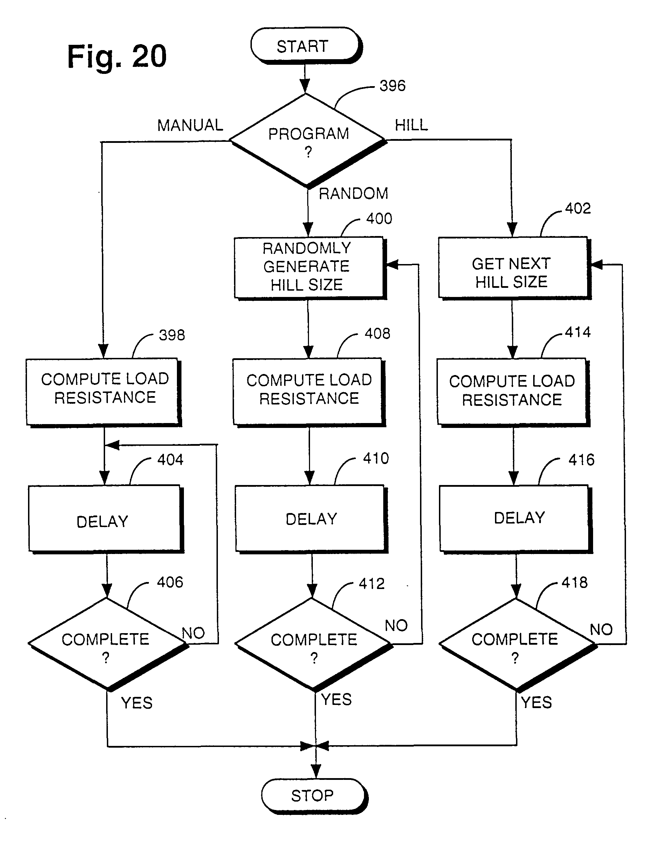

The invention may be best understood by describing the operation of exercise device 310 in the climbing mode. Initially, the user selects the climbing mode by pressing the B key. Software programs resident in non-volatile memory 358 of computer 354 are then executed to (i) prompt the user for various data, and (ii) perform an exercise program in accordance with the invention. Alternatively, the user could select the climbing mode after entering data or even during exercise, for example.
Referring to FIGS. 19 and 20, these software programs are now described. As shown at a block 386 of FIG. 19, computer 354 via panel 314 prompts the user to enter his weight in kilograms (pounds). At a block 388, the user is prompted to select one of the Hill, Random, and Manual programs by pressing one of the H, R and M keys, respectively.

At a block 390, the user is prompted to enter a completion criteria. The completion criteria establishes the basis for terminating the exercise in the Random and Manual programs. Suitable criteria include the elapsed time of exercise, the amount of calories expended, and the distance (expressed in floors) climbed.

In the case where the completion criteria is the amount of calories consumed, computer 352 calculates the calories consumed by the user on the basis of the load resistance, the distance climbed and the user's weight. Periodically, computer 352 compares the cumulative calories consumed to a user-entered target. When the actual cumulative calories consumed exceeds the target, the computer 352 alerts the user by an alarm, flashing light or other suitable means. Computer 352 can also discontinue load resistance to advise the user that the completion criteria has been reached.

Applicant's climbing mode allows the use of calorie consumption as a completion criteria because, unlike the prior art, the Applicant's climbing mode controls the load resistance during exercise.

At a block 392, the user is prompted to enter an effort level. In the preferred embodiment, there are twelve effort levels of increasing difficulty. Each level is associated with a range of load resistance, and the ranges may be successive or overlapping.

At a block 394, the exercise program is performed until the selected completion criteria is met or, in the case of the Hill Program, a predetermined routine is completed. Referring to FIG. 20, the operation of exercise device 310 at the block 394 is described in greater detail. Beginning at a block 396, computer 352 determines which of the Hill, Random and Manual programs the user has entered.

As discussed above, in accordance with the preferred embodiment of the invention, the exercise device 310 controls load resistance (as opposed to the rate of exercise), allowing the user to step at any desired rate in a more natural simulation of climbing. The resistance is varied in accordance with the selected one of the Hill, Random and Manual programs. In each case program, computer 352 selects one or more numbers (or "difficulty values") that are associated with hill height, size steepness or other indicator of difficulty. The load resistance is a function of these selected values. Thus, the higher the hill difficulty value, the greater the load resistance against the user's exercise movement. For purposes of this specification, the terms "hill height", "hill size" and "hill difficulty" are sometimes used interchangeably.

In the Hill program, the hill difficulty values are selected in accordance with a predetermined routine stored in nonvolatile memory 358. This routine is graphically displayed on an indicia 398 affixed to panel 314 (as seen in FIG. 18). The height of the vertical bars depicted in indicia 398 correspond to the amplitude of the load resistance with respect to time, which is represented along the horizontal axis, moving left to right. (The Hill program can also include a number of routines of varying but predetermined time durations.) A legend on the indicia 398 can associate the height of the vertical bars with the concept of hill height or difficulty.

In the Random program, the hill difficulty values are randomly selected from a predefined range by computer 352. In practice, the computer 352 can generate a series of random numbers when exercise begins, and can store the generated numbers in an array for serial retrieval during exercise. Once the random numbers are stored in memory, they become, in effect, a routine such as the predetermined routine invoked during operation of the Hill program.

In the Manual program, the load resistance is maintained at a fixed level of difficulty. Contrastingly, in the Hill and Random programs, the computer 358 successively selects (either randomly or in accordance with a preselected routine) one of (preferably) seven values of hill difficulty.

Referring again to FIG. 19, depending on which program has been selected, control moves from the block 396 to one of the blocks 398, 400, and 402. If the Manual program is selected, the computer 358 computes the load resistance at a block 398. After a delay period at block 404 (which may be of any suitable length such as for example one second) the computer 358 checks the completion criteria at a block 400 to determine if exercise is complete. If exercise is complete, the program terminates. Otherwise, control returns to the block 404, and the iteration of blocks 404 and 406 is repeated until the completion criteria is met.

If the Random program is selected, the computer 352 at a block 400 generates a random number corresponding to hill difficulty value. As mentioned, a series of random numbers may be generated at the same time and stored in an array. In this event, at the block 400 the computer 352 would fetch the next hill difficulty value from the array.

Alternatively, the computer can initially randomly generate a predetermined number of numbers, which are stored FIFO-wise in a circular buffer. With each iteration of the block 400, the earliest generated number is taken from the buffer, and a new randomly generated number is placed into the buffer. In this manner, at least some of the upcoming randomly selected hill difficulty values will be stored in memory for display on the program profile matrix 384, as described below.

The computer 352 then computes the load resistance at a block 408. After a delay period of any suitable length (represented by a block 410), the computer 354 checks the completion criteria at a block 412 to determine if exercise is complete. If exercise is complete, the program terminates. Otherwise, control returns to the block 400, and the iteration of blocks 400 and 408-412 is repeated until the completion criteria is met. In practice, a new difficulty value should be selected only on each Nth iteration or block 400, where N is a number sufficiently large so that the difficulty level does not change too often.

If the Hill program is selected, the computer 352 at a block 402 fetches the next hill difficulty value from the stored routine. The computer 352 then computes the load resistance at a block 414. After a delay period of any suitable length (represented by a block 416), computer 352 determines at block 117 if the predetermined routine is complete. If the preselected routine is complete, exercise terminates. Otherwise, control returns to the block 402, and the iteration of blocks 102 and 414-418 is repeated.

Load resistance as calculated by the computer 352 at the blocks 398, 408 and 414 is expressed as a duty cycle of the control signal 360 generated by the computer 352. Duty cycle is a computed function of the user's weight, the user-selected effort level and the current hill difficulty value (or "size"). In mathematical terms,$\text{Duty Cycle = F (weight, effort level, hill size).}$

In the Manual program, hill size (and, therefore, load resistance) is constant. But in the Hill and Random programs, hill size is varied, thus modulating load resistance over time.

Through empirical testing, a number of formulae have been found that can be used to compute duty cycle as a function of weight in kilograms (pounds), effort level and hill size. However, the following formula was found to be particularly effective:${\text{K}}_{\text{1}} {\text{* ( K}}_{\text{2}} {\text{+ weight * weight/K}}_{\text{3}} {\text{+ level * K}}_{\text{4}} {\text{+ [(level * K}}_{\text{5}} {\text{+ K}}_{\text{6}} {\text{) / K}}_{\text{7}} {\text{] * [hill - K}}_{\text{8}} \text{];}$ where K₁ eguals 0.33; K₂ equals 19; K₃ equals 2500; K₄ equals 2; K₅ equals 2; K₆ equals 30; K₇ equals 8; and K₈ equals 2. When the highest level is selected, K₄ may be set to three to give experienced users a particularly strenuous workout.

The actual values of the foregoing constants should be calibrated for specific embodiments of the invention. Of importance, however, is that the duty cycle be a function of the square of the user's weight. It is believed that the sensation of climbing is more realistic and comfortable when the weight term is thus squared.

The duty cycle as indicated above is generally proportional to the resistance felt by the user as he steps down on the pedals 316 and 326. This duty cycle as indicated by the formula above is computed independently of the user's rate of stepping as indicated by the display 372.

The program profile lamp matrix display 384 successively displays a plurality of images representing hills. Each hill image is associated with a difficulty level, which is graphically represented by the image. Specifically, display 384 indicates present and upcoming hill difficulty values in a matrix of LEDs 385. Each vertical column of matrix 384 contains successively illuminated LEDs 385 which define the image of a single "hill". The difficulty value or "height" of the hill is represented by the number of LEDs 385 that are illuminated in the particular column.

The current hill value is displayed in the far left column 420 (or "primary display"). Moving from left to right, the next N hill values which the user will encounter are displayed in display area 422 (or "secondary display"), where N is the number of remaining columns.

During each iteration of the Hill and Random routines, as a hill difficulty value is selected at the blocks 400 or 402 (as shown in FIG. 20), the selected hill value is displayed in the left-most column as the "current hill" value. Thus, the left-most column provides the user with a graphical, real-time representation of the level of load resistance which he or she is experiencing. The higher the displayed hill difficulty value, the greater the load resistance.

In this manner, the visual association of hill height in combination with the level of load resistance reinforces in the user's mind the connotation of steepness and the sensation of climbing hills. Alternatively, the invention may be practiced with more sophisticated displays, including three-dimensional or life-like video representations of hills.

The next N upcoming hill difficulty values displayed in area 422 are also updated each time a new hill value is selected by computer 352. In the Hill program, the computer 352 retrieves these values from the predetermined program. In the Random program, the computer 352 must generate and store at least N random hill values in advance if they are to be displayed in area 422.

It will be observed that the upcoming hill values appear to scroll from right to left across display area 422, providing further visual reinforcement of the sensation that the user is traversing a hilly terrain.

## Claims

1. An exercise apparatus comprising:
(a) movement means (28;44;50;316,318) adapted for engagement by the user for providing the user with an exercise movement, said movement means (28;44;50;316,318) being selectively adjustable for varying the intensity of exercise performed by the user;
(b) sensor means (38) adapted for selective engagement with the user for detecting a heart rate of the user and generating a signal indicating the user's level of exercise intensity based on said heart rate; and
(c) control means (30;46;51;314) operatively associated with said movement means (28;44;50;316,318) and said sensor means (38) for adjusting said movement means (28;44;50;316,318) in accordance with said signal to establish and maintain the user's exercise intensity near the target level;
**characterised by**
maintaining the user's level of exercise intensity near said target level during periods when the user has disengaged said sensor means (38) by maintaining the user's heart rate near a predetermined heart rate, including rate measuring means (64) for measuring the user's rate of exercise.

2. Exercise apparatus according to Claim 1,
**characterised by** including a display (32) operatively associated with said control means (30;46;51;314) for inviting the user to disengage said sensor means (38) for a time period once the user's level of exercise intensity reaches the target level.

3. Exercise apparatus according to Claims 1 or 2,
**characterised in that** said control means (30;46;51;314) is responsive to said rate measuring means (64) for maintaining the user's exercise intensity at or near the target level.

4. Exercise apparatus according to Claims 2 or 3,
**characterised in that** the duration of said time period is a function of the user's level of exercise intensity.

5. Exercise apparatus according to Claims 2, 3 or 4,
**characterised in that** said display (32) means includes prompting means (108) for prompting the user to reengage said sensor means (38) when said time period has expired.

6. Exercise apparatus according to Claims 1 to 5, a first and second pedal means (28;50;316,318) pivotally secured to a frame (26,48;312) and adapted for providing a user with a vertically reciprocating exercise movement;
resistance means for applying a variable level of resistance in opposition to said reciprocating exercise movement, said resistance means being selectively adjustable in response to a load control signal (66) for varying the level of resistance;
control means (30;46;51;314) for generating said control signal to apply said resistance independently of the rate of said exercise movement.

7. Exercise apparatus according to Claims 1 to 5,
**characterised in that** said movement means (28;44;50;316,318) comprises pedals (28), and said rate measuring means (64) measures the rate of exercise by measuring the rotational speed of said pedals (28).

8. Exercise apparatus according to Claims 1 to 5,
**characterised in that** said movement means (28;44;50;316,318) comprises a treadmill (22) having an endless belt (44), and said rate measuring means (64) measures the rate of exercise by measuring the speed of said endless belt (44).

9. Exercise apparatus according to Claims 1 to 5,
**characterised in that** said movement means (28;44;50;316,318) comprises a frame (312) having vertically reciprocating pedals (316,318) upon which the user stands to simulate stair climbing, and said rate measuring means (64) measures the rate of exercise by measuring the rate at which said pedals (316,318) reciprocate.

10. Exercise apparatus according to Claims 1 to 9,
**characterised by**
means for detecting whether the user is engaged with said sensor means (38); and alarm means (85) operatively associated with said detecting means for alerting the user if the user has not reengaged said sensor means (38) within a predetermined amount of time after said time period has expired.

## Patentansprüche

1. Trainingsgerät, welches folgendes aufweist:
(a) eine Bewegungseinrichtung (28;44;50;316;,318), welche so ausgebildet ist, daß sie bei einem Eingriff durch den Benutzer eine Trainingsbewegung für den Benutzer schafft, wobei die Bewegungseinrichtung (28;44;50;316,318) selektiv einstellbar ist, um die Trainingsintensität für den Benutzer zu variieren;
(b) eine Sensoreinrichtung (38) welche dafür vorgesehen ist, selektiv mit dem Benutzer in Eingriff zu stehen zum Ermitteln eines Pulses des Benutzers und Erzeugen eines Signals, welche den Grad der Trainingsintensität des Benutzers basierend auf dem Puls anzeigt; und
(c) eine Steuereinrichtung (30;46;51;314) welche im Betrieb mit der Bewegungseinrichtung (28;44;50;316,318) und der Sensoreinrichtung (38) in Verbindung steht zum Einstellen der Bewegungseinrichtung (28;44;50;316,318) in Übereinstimmung mit dem Signal, um die Trainingsintensität des Benutzers in die Nähe des Zielniveaus zu bringen und dort zu halten,
**gekennzeichnet durch**
das Halten des Niveaus der Trainingsintensität des Benutzers während Zeitabschnitten in der Nähe des Zielniveaus, wenn der Benutzer die Sensoreinrichtung (38) außer Eingriff gebracht hat **durch** Beibehalten des Pulses des Benutzers in der Nähe eines voreingestellten Pulses, einschließlich einer Niveaumeßeinrichtung (64) zum Messen der Trainingsrate des Benutzers.

2. Trainingsgerät nach Anspruch 1,
**gekennzeichnet durch**
ein Display (32), welches betriebsfähig mit der Steuereinrichtung (30;46;51;314) verbunden ist, um den Benutzer dazu aufzufordern, die Sensoreinrichtung (38) für eine Zeitdauer außer Eingriff zu bringen, sobald das Niveau der Trainingsintensität des Benutzers das Zielniveau erreicht.

3. Trainingsgerät nach Anspruch 1 oder 2,
**dadurch gekennzeichnet, daß**
die Steuereinrichtung (30;46;51;314) auf die Niveaumeßeinrichtung (64) reagiert, um die Trainingsintensität des Benutzers an oder in der Nähe des Zielniveaus zu halten.

4. Trainingsgerät nach Anspruch 2 oder 3,
**dadurch gekennzeichnet, daß**
die Länge der Zeitdauer eine Funktion des Niveaus der Trainingsintensität des Benutzers ist.

5. Trainingsgerät nach Anspruch 2, 3 oder 4,
**dadurch gekennzeichnet, daß**
das Display (32) eine Veranlassungs-Einrichtung (108) zum Veranlassen des Benutzers, die Sensoreinrichtung (38) wieder in Eingriff zu bringen, wenn die Zeitdauer abgelaufen ist, aufweist.

6. Trainingsgerät nach einem der Ansprüche 1 bis 5,
mit einer ersten und zweiten Pedaleinrichtung (28;50;316,318), welche gelenkig an einem Rahmen (26,48;312) angebracht und dafür vorgesehen ist, dem Benutzer eine sich in vertikaler Richtung wiederholende Trainingsbewegung anzubieten;
eine Widerstandseinrichtung zum Aufbringen eines variablen Widerstandsniveaus entgegen der sich wiederholenden Trainingsbewegung, wobei die Widerstandseinrichtung selektiv einstellbar als Reaktion auf ein Belastungssteuersignal (66) zum Verändern des Widerstandsniveaus ist;
eine Steuereinrichtung (30;46;51;314) zum Erzeugen des Steuersignals, um den Widerstand unabhängig von dem Niveau der Trainingsbewegung aufzubringen.

7. Trainingsgerät nach einem der Ansprüche 1 bis 5,
**dadurch gekennzeichnet, daß**
die Bewegungseinrichtung (28;44;50;316,318) Pedale (28) aufweist, und daß die Niveaumeßeinrichtung (64) das Trainingsniveau durch Messen der Rotationsgeschwindigkeit der Pedale (28) mißt.

8. Trainingsgerät nach einem der Ansprüche 1 bis 5,
**dadurch gekennzeichnet, daß**
die Bewegungseinrichtung (28;44;50;316,318) ein Laufband (22) mit einem endlosen Band (44) aufweist, und daß die Niveaumeßeinrichtung (64) das Trainingsniveau durch Messen der Geschwindigkeit des endlosen Bandes (44) mißt.

9. Trainingsgerät nach einem der Ansprüche 1 bis 5,
**dadurch gekennzeichnet, daß**
die Bewegungseinrichtung (28;44;50;316,318) einen Rahmen (312) aufweist, welcher in vertikaler Richtung sich auf- und abbewegende Pedale (316,318) aufweist, auf welchen der Benutzer zur Simulation von Treppensteigen steht, und daß die Niveaumeßeinrichtung (64) das Trainingsniveau durch Messen der Rate, um welche sich die Pedale (316,318) auf- und abbewegen, mißt.

10. Trainingsgerät nach einem der Ansprüche 1 bis 9,
**gekennzeichnet durch,**
eine Einrichtung zur Ermittlung, ob der Benutzer in Eingriff mit der Sensoreinrichtung (38) steht; und eine Alarmeinrichtung (85), welche beriebsfähig mit der Ermittlungseinrichtung verbunden ist, zum Alarmieren des Benutzers, wenn der Benutzer die Sensoreinrichtung (38) nicht innerhalb einer vorbestimmten Zeitdauer nachdem die Zeitdauer abgelaufen ist wieder in Eingriff gebracht hat

## Revendications

1. Appareil d'exercice comprenant :
(a) des moyens de mouvement (28; 44; 50; 316, 318) agencés pour que l'usager puisse s'y installer pour fournir un exercice physique, lesdits moyens de mouvement (28; 44; 50; 316, 318) étant sélectivement réglables pour faire varier l'intensité de l'exercice exécuté par l'usager;
(b) des moyens de détection (38) agencés pour être reliés à l'usager selon le besoin, pour détecter son rythme cardiaque et produire un signal indiquant le niveau d'intensité de l'exercice physique de l'usager en fonction dudit rythme cardiaque; et
(c) des moyens de contrôle (30; 46; 51; 314) reliés de manière fonctionnelle auxdits moyens de mouvement (28; 44; 50; 316, 318) et auxdits moyens de détection (38) pour ajuster lesdits moyens de mouvement (28; 44; 50; 316, 318) en fonction dudit signal pour établir et maintenir l'intensité d'exercice physique de l'usager proche du niveau visé;
**caractérisé par**
le maintien du niveau d'intensité d'exercice physique de l'usager proche dudit niveau visé, pendant des périodes durant lesquelles l'usager n'est pas relié aux moyens de détection (38), en maintenant le rythme cardiaque de l'usager proche d'un rythme cardiaque prédéterminé, et comprenant des moyens de mesure (64) pour mesurer le rythme d'exercice de l'usager.

2. Appareil d'exercice selon la revendication 1,
**caractérisé par**
l'adjonction d'un système d'affichage (32) associé de façon opérationnelle auxdits moyens de contrôle (30; 46; 51; 314) pour inviter l'usager à se déconnecter desdits moyens de détection (38) pendant une certaine période de temps une fois que son niveau d'intensité d'exercice physique a atteint le niveau visé.

3. Appareil d'exercice selon les revendications 1 ou 2,
**caractérisé en ce que,**
lesdits moyens de contrôle (30; 46; 51; 314) sont associés auxdits moyens de mesure de rythme (64) pour maintenir l'intensité de l'exercice physique de l'usager au niveau ou proche du niveau visé.

4. Appareil d'exercice selon les revendications 2 ou 3,
**caractérisé en ce que**,
la durée de ladite période de temps est fonction de l'intensité de l'exercice physique de l'usager.

5. Appareil d'exercice selon les revendications 2, 3 ou 4,
**caractérisé en ce que**
ledit système d'affichage (32) comprend un indicateur (108) pour inciter l'usager à se reconnecter auxdits moyens de détection (38) lorsque ladite période de temps est écoulée.

6. Appareil d'exercice selon les revendications 1 à 5, comportant
une première et une seconde pédale (28; 50; 316, 318) fixées de manière pivotante à un cadre (26, 48; 312) et agencées pour que l'usager puisse effectuer un mouvement de va et vient vertical d'exercice physique;
des moyens de résistance permettant d'appliquer une résistance de niveau variable s'opposant audit mouvement de va-et-vient, lesdits moyens de résistance étant sélectivement réglables, en réponse à un signal de contrôle de charge (66), pour faire varier le niveau de résistance;
des moyens de contrôle (30; 46; 51; 314) pour produire ledit signal de contrôle à appliquer à ladite résistance indépendamment du rythme dudit mouvement d'exercice phvsique.

7. Appareil d'exercice selon les revendications 1 à 5,
**caractérisé en ce que**,
lesdits moyens de mouvement (28; 44; 50; 316, 318) comprennent des pédales (28) et lesdits moyens de mesure de rythme (64) mesurent le rythme de l'exercice en mesurant la vitesse de rotation desdites pédales (28).

8. Appareil d'exercice selon les revendications 1 à 5,
**caractérisé en ce que**
lesdits moyens de mouvement (28; 44; 50; 316, 318) comprennent un tapis de jogging (22) comportant une courroie sans fin (44), et lesdits moyens de mesure de rythme (64) mesurent le rythme de l'exercice physique en mesurant la vitesse de ladite courroie sans fin (44).

9. Appareil d'exercice selon les revendications 1 à 5,
**caractérisé en ce que**
lesdits moyens de mouvement (28; 44; 50; 316,18) comprennent un cadre (312) comportant des pédales ayant un mouvement vertical de va-et-vient (316, 318) sur lesquelles l'usager se tient pour simuler la montée d'escaliers, et lesdits moyens de mesure de rythme (64) mesurent le rythme de l'exercice physique en mesurant le rythme auquel lesdites pédales (316, 318) vont et viennent.

10. Appareil d'exercice selon les revendications 1 à 9,
**caractérisé par**
des moyens pour détecter si l'usager est connecté auxdits moyens de détection (38); et un système d'alarme (85) associé de façon opérationnelle auxdits moyens de détection pour prévenir l'usager si celui-ci ne s'est pas reconnecté auxdits moyens de détection (38) à l'intérieur d'un intervalle de temps prédéterminé lorsque ladite période de temps est écoulée.
